# EUROPEAN PATENT APPLICATION

(11) **EP 1 262 550 A2**
(43) Date of publication of application: **04.12.2002**
(21) Application number: 02011881.6
(22) Date of filing: 28.05.2002
(51) Int. Cl.: C12N 15/53, C12N 9/04, C12P 7/18, C12P 41/00, C12P 7/26

(54) **(R)-2,3-butanediol dehydrogenase, methods for producing same, and methods for producing optically active alcohol using the dehydrogenase**

(30) Priority: 28.05.2001 JP 2001159647
(71) Applicant: Daicel Chemical Industries, Ltd., Osaka 590-8501 (JP)
(72) Inventor: Yamamoto, Hiroaki, Tsukuba-shi, Ibaraki 305-0047 (JP); Kimoto, Norihiro, Tsukuba-shi, Ibaraki 305-0047 (JP)
(74) Representative: Grünecker, Kinkeldey, Stockmair & Schwanhäusser Anwaltssozietät

(57) **Abstract**

The object of the present invention is to provide an (R)-2,3-butanediol dehydrogenase which uses NADH as a coenzyme, and methods for producing optically active alcohols and ketones using the enzyme. The inventors of the present invention discovered a novel (R)-2,3-butanediol dehydrogenase, isolated a DNA encoding the dehydrogenase, and produced recombinants that express the dehydrogenase at high levels. The dehydrogenase is produced by and can be isolated and purified from *Kluyveromyces lactis.* The use of the dehydrogenase of the invention enables efficient production of (R)-1,3-butanediol with high optical purity from 4-hydroxy-2-butanone. Also provided by the present invention are methods for efficiently producing (S)-1,3-butanediol with high optical purity from racemic 1,3-butanediol, as well as 4-hydroxy-2-butanone from (R)-1,3-butanediol.

SEQUENCE LISTING

<110> Daicel Chemical Industries, Co., Ltd.

<120> NOVEL (R)-2,3-BUTANEDIOL DEHYDROGENASE, METHODS FOR PRODUCING SAME,
AND METHODS FOR PRODUCING OPTICALLY ACTIVE ALCOHOL USING THE DEHYDROGENASE

<130> D1-A0105

<140>
<141>

<150> JP 2001-159647
<151> 2001-05-28

<160> 11

<170> PatentIn Ver. 2.1

<210> 1
<211> 1158
<212> DNA
<213> Kluyveromyces lactis

<400> 1
atgcgtgcat tagcttattt cggaaaacaa gacatcagat acacaaagga tttggaggaa 60
cctgtcatcg aaacagatga tggaattgaa atcgaagtct catggtgtgg tatctgcggt 120
agtgatttac acgaatacct agatggtcct attttctttc cagaagatgg caaggtccac 180
gatgttagtg gtcttggatt gcctcaagct atgggtcatg agatgtccgg tatcgtatca 240
aaagttggac caaaagtaac caacatcaag gctggtgatc atgttgttgt agaagccacc 300
ggtacatgtc ttgatcatta cacttggcct aacgctgcac atgctaagga tgctgaatgt 360
gctgcttgtc aaagagggtt ctacaactgt tgtgcccatt tgggtttcat gggtctagga 420
gttcacagtg gtggttttgc tgaaaaagtc gttgttagtg aaaagcacgt tgttaagatt 480
ccaaacactt taccattgga cgttgcagcc ttagtcgaac caatctccgt ctcatggcat 540
gctgttagaa tctccaagtt acagaagggc caatccgcct tagttcttgg cgcaggtcca 600
attggattag ccaccatttt agctttgcaa ggtcatggtg cctccaagat tgtagtctct 660
gaaccagctg aaatcagaag aaatcaagct gccaagcttg gtgtcgaaac cttcgatcct 720

tctgaacata aagaagatgc cgttaatatt ttgaagaaat tggctccagg tggtgaaggt 780
ttcgattttg cctacgattg ttctggtgtt aaaccaactt ttgatacagg tgtccatgct 840
actaccttca gaggtatgta tgttaatatt gcaatctggg gccacaaacc catcgatttc 900
aaacctatgg acgtcactct gcaagagaaa ttcgtcaccg gttccatgtg ttacaccatt 960
aaagattttg aagatgtggt ccaagcctta ggcaatggca gcattgctat tgataaagct 1020
agacatttga ttacgggtag acaaaaaatc gaagatgggt tcactaaagg gttcgacgaa 1080
ttaatgaacc ataaagagaa aaacatcaag atattgttga ctcctaataa tcatggtgag 1140
ttggatgcca ccaactga 1158

<210> 2
<211> 385
<212> PRT
<213> Kluyveromyces lactis

<400> 2
Met Arg Ala Leu Ala Tyr Phe Gly Lys Gln Asp Ile Arg Tyr Thr Lys
1               5                   10                  15

Asp Leu Glu Glu Pro Val Ile Glu Thr Asp Asp Gly Ile Glu Ile Glu
            20                  25                  30

Val Ser Trp Cys Gly Ile Cys Gly Ser Asp Leu His Glu Tyr Leu Asp
        35                  40                  45

Gly Pro Ile Phe Phe Pro Glu Asp Gly Lys Val His Asp Val Ser Gly
    50                  55                  60

Leu Gly Leu Pro Gln Ala Met Gly His Glu Met Ser Gly Ile Val Ser
65                  70                  75                  80

Lys Val Gly Pro Lys Val Thr Asn Ile Lys Ala Gly Asp His Val Val
                85                  90                  95

Val Glu Ala Thr Gly Thr Cys Leu Asp His Tyr Thr Trp Pro Asn Ala
            100                 105                 110

Ala His Ala Lys Asp Ala Glu Cys Ala Ala Cys Gln Arg Gly Phe Tyr
        115                 120                 125

Asn Cys Cys Ala His Leu Gly Phe Met Gly Leu Gly Val His Ser Gly
    130                 135                 140

Gly Phe Ala Glu Lys Val Val Val Ser Glu Lys His Val Val Lys Ile
145                 150                 155                 160

Pro Asn Thr Leu Pro Leu Asp Val Ala Ala Leu Val Glu Pro Ile Ser
                165                 170                 175

Val Ser Trp His Ala Val Arg Ile Ser Lys Leu Gln Lys Gly Gln Ser
            180                 185                 190

Ala Leu Val Leu Gly Ala Gly Pro Ile Gly Leu Ala Thr Ile Leu Ala
        195                 200                 205

Leu Gln Gly His Gly Ala Ser Lys Ile Val Val Ser Glu Pro Ala Glu
    210                 215                 220

Ile Arg Arg Asn Gln Ala Ala Lys Leu Gly Val Glu Thr Phe Asp Pro
225                 230                 235                 240

Ser Glu His Lys Glu Asp Ala Val Asn Ile Leu Lys Lys Leu Ala Pro
                245                 250                 255

Gly Gly Glu Gly Phe Asp Phe Ala Tyr Asp Cys Ser Gly Val Lys Pro
            260                 265                 270

Thr Phe Asp Thr Gly Val His Ala Thr Thr Phe Arg Gly Met Tyr Val
        275                 280                 285

Asn Ile Ala Ile Trp Gly His Lys Pro Ile Asp Phe Lys Pro Met Asp
    290                 295                 300

Val Thr Leu Gln Glu Lys Phe Val Thr Gly Ser Met Cys Tyr Thr Ile

305                 310                 315                 320

Lys Asp Phe Glu Asp Val Val Gln Ala Leu Gly Asn Gly Ser Ile Ala
                325                 330                 335

Ile Asp Lys Ala Arg His Leu Ile Thr Gly Arg Gln Lys Ile Glu Asp
            340                 345                 350

Gly Phe Thr Lys Gly Phe Asp Glu Leu Met Asn His Lys Glu Lys Asn
        355                 360                 365

Ile Lys Ile Leu Leu Thr Pro Asn Asn His Gly Glu Leu Asp Ala Thr
    370                 375                 380

Asn
385

<210> 3
<211> 10
<212> PRT
<213> Kluyveromyces lactis

<400> 3
Met Arg Ala Leu Ala Tyr Phe Gly Lys Gln
  1               5                  10

<210> 4
<211> 10
<212> PRT
<213> Kluyveromyces lactis

<400> 4
Leu Ala Pro Gly Gly Glu Gly Phe Asp Phe
  1               5                  10

<210> 5
<211> 29
<212> DNA
<213> Artificial Sequence

<220>
<223> Description of Artificial Sequence:an artificially
      synthesized primer sequence

<220>
<221> misc_feature
<222> (15)
<223> n indicates g, a, c or t.

<220>
<221> misc_feature
<222> (27)
<223> n indicates g, a, c, or t.

<400> 5
gtcggatccg chytngchta yttyggnaa                                    29

<210> 6
<211> 30
<212> DNA
<213> Artificial Sequence

<220>
<223> Description of Artificial Sequence:an artificially
      synthesized primer sequence

<400> 6
cagggatccr aartcraadc cytcdccdcc                                   30

<210> 7
<211> 745
<212> DNA
<213> Kluyveromyces aestuarii

<400> 7
aacaagacat cagatacaca aaggatttgg aggaacctgt catcgaaaca gatgatggaa 60
ttgaaatcga agtctcatgg tgtggtatct gcggtagtga tttacacgaa tacctagatg 120
gtcctatttt ctttccagaa gatggcaagg tccacgatgt tagtggtctt ggattgcctc 180
aagctatggg tcatgagatg tccggtatcg tatcaaaagt tggaccaaaa gtaaccaaca 240
tcaaggctgg tgatcatgtt gttgtagaag ccaccggtac atgtcttgat cattacactt 300
ggcctaacgc tgcacatgct aaggatgctg aatgtgctgc ttgtcaaaga gggttctaca 360
actgttgtgc ccatttgggt ttcatgggtc taggagttca cagtggtggt tttgctgaaa 420
aagtcgttgt tagtgaaaag cacgttgtta agattccaaa cactttacca ttggacgttg 480
cagccttagt cgaaccaatc tccgtctcat ggcatgctgt tagaatctcc aagttacaga 540
agggccaatc cgccttagtt cttggcgcag gtccaattgg attagccacc attttagctt 600
tgcaaggtca tggtgcctcc aagattgtag tctctgaacc agctgaaatc agaagaaatc 660
aagctgccaa gcttggtgtc gaaaccttcg atccttctga acataaagaa gatgccgtta 720
atattttgaa gaaattggct ccagg 745

<210> 8
<211> 29
<212> DNA
<213> Artificial Sequence

<220>
<223> Description of Artificial Sequence:an artificially
synthesized primer sequence

<400> 8
cagggatcca ccgcagatac cacaccatg 29

<210> 9
<211> 29
<212> DNA

<213> Artificial Sequence

<220>
<223> Description of Artificial Sequence:an artificially
      synthesized primer sequence

<400> 9
gtcggatccg tgtcgaaacc ttcgatcct                                    29


<210> 10
<211> 36
<212> DNA
<213> Artificial Sequence

<220>
<223> Description of Artificial Sequence:an artificially
      synthesized primer sequence

<400> 10
cttgaattct accatgcgtg cattagctta tttcgg                            36


<210> 11
<211> 45
<212> DNA
<213> Artificial Sequence

<220>
<223> Description of Artificial Sequence:an artificially
      synthesized primer sequence

<400> 11
cagcttaaga tctctagatt agttggtggc atccaactca ccatg                  45

## Description

### FIELD OF THE INVENTION

The present invention relates to a novel NAD⁺-dependent (R)-2,3-butanediol dehydrogenase. The present invention also relates to polynucleotides encoding the enzyme protein (dehydrogenase), as well as methods for producing the enzyme, methods for producing optically active alcohols, particularly (R)-1,3-butanediol or (S)-1,3-butanediol, using recombinants that express the enzyme, and methods for producing ketones, particularly 4-hydroxy-2-butanone.

### BACKGROUND OF THE INVENTION

Optically active 1,3-butanediol is a useful compound that serves as a raw material for synthetic intermediates of antibiotics, such as azetidinone (J. Chem. Soc., Chem. Commun., 9, 662-664 (1991)). Methods for producing optically active 1,3-butanediol known in the art include:
(1) a method wherein chemically synthesized racemic 1,3-butanediol is optically resolved using an optical resolution agent (Unexamined Published Japanese Patent Application No. (JP-A) Sho 61-191631) ;
(2) asymmetric synthesis from 4-hydroxy-2-butanone using Raney nickel catalyst treated with optically active compounds (JP-A Sho 58-204187; and Bull. Chem. Soc. Jpn., 53, 1356-1360 (1980));
(3) a method wherein racemic 1,3-butanediol is reacted with microorganisms to remain (R)-1,3-butanediol (Examined Published Japanese Patent Application No. (JP-B) Hei 6-95951; and JP-A Hei 7-231785);
(4) a method wherein (R)-1,3-butanediol is produced by an asymmetric reduction reaction by contacting microorganisms with 4-hydroxy-2-butanone (Japanese Patent No. 2731589); and
(5) a method wherein racemic 1,3-butanediol is reacted with the microorganisms to remain (S)-1,3-butanediol (JP-A Hei 7-39390).

However, the methods of (1) and (2) require the use of expensive optical resolution agents or catalysts. In addition, the methods of (2), (3), (4), and (5) have a poor reaction yield, low optical purity, and other disadvantages.

With respect to enzymes having 2,3-butanediol dehydrogenation activity, according to a research on biosynthesis and metabolism of 2,3-butanediol (Arch. Microbiol. 116, 197-203, 1978; J. Ferment. Technol. 61, 467-471, 1983; J. Ferment. Technol. 62, 551-559, 1984), dehydrogenase activity on (2R,3R)-2,3-butanediol has been reported in, for example, the microorganisms listed below. However, since the activity had been measured only using cell-free extracts composed of various enzymes, the stereoselectivity and other properties of 2,3-butanediol dehydrogenase produced thereby have not yet been clearly determined.
*Aeromonas hydrophila*
*Bacillus cereus* IAM 1072
*Bacillus coagulans* ATCC 8038
*Micrococcus lysodeikticus* IAM 1056
*Micrococcus luteus* IAM 1097
*Micrococcus roseus* IAM 1295
*Pseudomonas saccharophila* IAM 1504
*Sarcina lutea* IAM 1099
*Staphylococcus aureus*

In addition, enzymes such as below have been shown to possess a 2,3-butanediol dehydrogenase activity among highly purified enzymes with determined properties. However, the activity of these enzymes has been reported only for the DL conformation and their stereoselectivity remains unknown.
Glycerol dehydrogenase derived from *Achromobacter liquidum* KY3047 (JP-B Sho 58-40467)
Glycerol dehydrogenase derived from *Bacillus* sp. G-1 (JP-B Hei 03-72272)
Glycerol dehydrogenase derived from *Bacillus* *stearothermophilus* (Biochim. Biophys. Acta 994, 270-279 (1989))
Glycerol dehydrogenase derived from *Citrobacter freundii* DSM 30040 (J. Bacteriol. 177, 4392-4401 (1995))
Glycerol dehydrogenase derived from *Erwinia aroideae* IFO 3830 (Chem. Pharm. Bull. 26, 716-721 (1978))
Glycerol dehydrogenase derived from *Geotrichum candidum* IFO 4597 (JP-B Hei 01-27715)
Dihydroxyacetone reductase derived from *Pichia ofunaensis* AKU 4328 (J. Biosci. Bioeng. 88, 148-152 (1999))
Glycerol dehydrogenase derived from *Schizosaccharomyces pombe* (J. Gen. Microbiol. 131, 1581-1588 (1985))
Glycerol dehydrogenase produced from *Escherlchia coli* W-1485 (J. Biol. Chem. 259, 2124-2129 (1984)) is an example of a highly purified enzyme with a determined high selectivity for the (2R,3R) conformation of 2,3-butanediol. The Vmax of the enzyme relative to (2R,3R)-2,3-butanediol is 28.0 U/mg protein and that relative to its racemate is 21.2 U/mg protein, which suggests the stereoselectivity for the (2R,3R) conformation of the enzyme. Herein, 1 U of enzyme refers to an enzyme activity that reduces 1 µmol of NAD⁺ to NADH in one minute using (2R,3R)-2,3-butanediol as substrate.

The molecular weight of the enzyme determined by SDS-PAGE is 55,000, while that by gel filtration is 417,000, which suggest the enzyme exists in the form of an octamer. Further, the enzyme is activated by ammonium ion and the optimum pH for the oxidation reaction is in the vicinity of 10 (J. Biol. Chem. 259, 2124-2129 (1984)), which distinguishes the enzyme from the enzyme of the present invention. Furthermore, the stereoselectivity of this enzyme for 1,3-butanediol has not been reported.

Although (R)-2,3-butanediol dehydrogenase derived from *Saccharomyces cerevisiae* (Arch. Microbiol. 154, 267-273 (1990)) has been reported to produce (2R,3R)-2,3-butanediol from 2,3-butanedione, asymmetric reduction of 4-hydroxy-2-butanone have not yet been reported.

Finally, a gene, encoding 2,3-butanediol dehydrogenase involved in 2,3-butanediol metabolism, has been cloned from *Pseudomonas putida.* Although this enzymes has been expressed in *Escherichia coli* (FEMS Microbiol. Lett. 124(2), 141-150 (1994)), the stereoselectivity of the enzyme has not been reported. Further, a gene with high homology to the 2,3-butanediol dehydrogenase gene derived from *Pseudomonas putida* has been identified as a result of genome analysis of *Pseudomonas aeroginosa.* However, the activity, stereoselectivity, and such of the enzyme encoded by this gene have not been determined.

Thus, a method for obtaining optically active 1,3-butanediol of high optical purity with a high reaction yield that is both economically superior and simple is needed.

### SUMMARY OF THE INVENTION

An object of the present invention is to provide methods for producing optically active 1,3-butanediol of high optical purity with an efficient yield.

Further, another object of the present invention is to provide a novel enzyme that produces (R)-1,3-butanediol of high optical purity by reducing 4-hydroxy-2-butanone using NADH as the coenzyme. Moreover, another object of the present invention is to obtain a recombinant by isolating a polynucleotide encoding the enzyme with desired properties. Furthermore, another object of the present invention is to provide a method for producing optically active 1,3-butanediol using the recombinant.

The inventors of the present invention focused on the selectivity of the enzyme to obtain optically active 1,3-butanediol of high optical purity by methods that are both economically superior and simple. For example, the above problems can be solved by finding an enzyme which produces (R)-1,3-butanediol by stereoselectively reducing 4-hydroxy-2-butanone. Moreover, an efficient production of (R)-1,3-butanediol from 4-hydroxy-2-butanone using a gene recombinant microorganism is enabled by expressing such enzymes at high levels in heterologous microorganisms.

As a result of a search for such enzymes, the inventors of the present invention discovered an enzyme having superior reaction yield and high stereoselectivity, and succeeded in purifying the enzyme. The enzyme is a novel (R)-2,3-butanediol dehydrogenase that has both high activity and high stereoselectivity for dehydrogenating the hydroxyl group of the (R) configuration of 2,3-butanediol.

The enzyme of the present invention produces (R)-1,3-butanediol of high optical purity at a high yield by reducing 4-hydroxy-2-butanone in a reduction reaction. Further, the present enzyme produces (S)-1,3-butanediol and 4-hydroxy-2-butanone of high optical purity by specifically oxidizing (R)-1,3-butanediol of racemic 1,3-butanediol in an oxidation reaction. The stereoselectivity of the (R)-2,3-butanediol dehydrogenase of the present invention is distinct in that the optical purity of the (R)-1,3-butanediol produced by the reduction of 4-hydroxy-2-butanone is normally 90% ee (enatiomeric excess)or more, and desirably 99% *ee* or more.

Furthermore, the inventors of the present invention succeeded in isolating a DNA encoding this enzyme and constructing a recombinant that highly expresses this enzyme. The inventors of the present invention also established a novel method for producing optically active alcohols or ketones using this enzyme or the enzyme activity of recombinants, and completed the present invention. More specifically, the present invention relates to the following (R)-2,3-butanediol dehydrogenase, and polynucleotides encoding the same, as well as production methods and uses of the enzyme:
[1] an (R)-2,3-butanediol dehydrogenase having the following physicochemical properties:
   (1) Action:
      the enzyme acts on (2R,3R)-2,3-butanediol, using nicotinamide adenine dinucleotide (hereinafter, abbreviated as NAD⁺) as a coenzyme, to produce (R)-acetoin. The enzyme reduces 2,3-butanedione, using the reduced form of nicotinamide adenine dinucleotide (hereinafter, abbreviated as NADH) as a coenzyme, to produce (2R,3R)-2,3-butanediol;
   (2) Substrate specificity:
      (a) the enzyme uses NAD⁺ as a coenzyme in the oxidation reaction. It uses NADH as a coenzyme in the reduction reaction;
      (b) the enzyme reduces 4-hydroxy-2-butanone to produce (R)-1,3-butanediol;
      (c) the enzyme preferentially oxidizes the hydroxyl group of the (R) configuration of racemic 1,3-butanediol to remain the (S)-1,3-butanediol; and
   (3) Optimal pH:
      pH 6.0 for both of the oxidation reaction and the reduction reaction;
[2] the (R)-2,3-butanediol dehydrogenase of [1] further having the following additional physicochemical properties:
   (4) Optimum temperature: 37°C for both of the oxidation reaction and reduction reaction; and
   (5) Molecular weight:
      the molecular weight of the enzyme determined by sodium dodecyl sulfate-polyacrylamide gel electrophoresis (hereinafter, abbreviated as SDS-PAGE) and by gel filtration is 45,000 and 91,000, respectively;
[3] the (R)-2,3-butanediol dehydrogenase of [1] which is produced from a microorganism belonging to the genus *Kluyveromyces*;
[4] the (R)-2,3-butanediol dehydrogenase of [3], wherein the microorganism belonging to the genus *Kluyveromyces* is *Kluyveromyces lactis;*
[5] a polynucleotide selected from the group consisting of:
   (a) a polynucleotide comprising the nucleotide sequence of SEQ ID NO: 1;
   (b) a polynucleotide encoding a polypeptide comprising the amino acid sequence of SEQ ID NO: 2;
   (c) a polynucleotide encoding a polypeptide comprising the amino acid sequence of SEQ ID NO: 2 in which one or more amino acids are substituted, deleted, inserted, and/or added, wherein said polypeptide is functionally equivalent to a polypeptide consisting of the amino acid sequence of SEQ ID NO: 2;
   (d) a polynucleotide hybridizing under stringent conditions with a DNA consisting of the nucleotide sequence of SEQ ID NO: 1, wherein the polypeptide encoded by the polynucleotide is functionally equivalent to the polypeptide consisting of the amino acid sequence of SEQ ID NO: 2; and
   (e) a polynucleotide encoding an amino acid sequence having 80% or more identity with the amino acid sequence of SEQ ID NO: 2;
[6] a polypeptide encoded by the polynucleotide of [5];
[7] a vector comprising the polynucleotide of [5];
[8] a transformant retaining the polynucleotide of [5] or the vector of [7];
[9] a method for producing the polypeptide of [6], comprising the steps of culturing the transformant of [8], and recovering the expressed product thereof;
[10] a method for producing the enzyme of [1] or the polypeptide of [6], comprising the step of culturing a microorganism belonging to the genus *Kluyveromyces*, that produces the enzyme of [1] or the polypeptide of [6];
[11] the method of [10], wherein the microorganism belonging to the genus *Kluyveromyces* is *Kluyveromyces lactis;*
[12] a method for producing an optically active alcohol, comprising the steps of: reacting a ketone in the presence of NADH with any enzyme active substance selected from the group consisting of: (a) the (R)-2,3-butanediol dehydrogenase of [1], (b) the polypeptide of [6], (c) a microorganism producing (a) or (b), and (d) treated or processed products of (a), (b) or (c); and obtaining the optically active alcohol produced by the reduction of the ketone;
[13] the method of [12], wherein the microorganism is the transformant of [8];
[14] the method of [12], wherein the ketone is 4-hydroxy-2-butanone, and the optically active alcohol is (R)-1,3-butanediol;
[15] a method for producing optically active alcohols, comprising the steps of reacting a racemic alcohol in the presence of NAD⁺ with any enzyme active substance selected from the group consisting of: (a) the (R)-2,3-butanediol dehydrogenase of [1], (b) the polypeptide of [6], (c) a microorganism that produces (a) or (b), and (d) processed products of (a) to (c) to oxidize one of the optical isomers; and obtaining the remaining optically active alcohol;
[16] the method of [15], wherein the microorganism is the transformant of [8] ;
[17] the method of [15], wherein the racemic alcohol is racemic 1,3-butanediol, and the optically active alcohol is (S)-1,3-butanediol;
[18] a method for producing ketones comprising the steps of contacting an alcohol in the presence of NAD⁺ with any enzyme active substance selected from the group consisting of: (a) the (R)-2,3-butanediol dehydrogenase of [1], (b) the polypeptide of [6], (c) a microorganism that produces (a) or (b), and (d) processed products of (a) to (c); and obtaining the ketone produced by the oxidation of the alcohol;
[19] the method of [18], wherein the microorganism is the transformant of [8];
[20] the method of [18], wherein the alcohol is (R)-1,3-butanediol, and the ketone is 4-hydroxy-2-butanone;
[21] a method for producing (R)-1,3-butanediol, comprising the steps of:
   (1) producing (R)-1,3-butanediol and 4-hydroxy-2-butanone by preferentially oxidizing (S)-1,3-butanediol by reacting racemic 1,3-butanediol with any enzyme active substance selected from the group consisting of: an enzyme that produces 4-hydroxy-2-butanone, a microorganism producing the enzyme, and processed products of the enzyme or microorganism;
   (2) producing (R)-1,3-butanediol by reducing 4-hydroxy-2-butanone by contacting the 4-hydroxy-2-butanone produced in step (1) with any enzyme active substance selected from the group consisting of: an enzyme that produces (R)-1,3-butanediol, a microorganism producing the enzyme, and processed products of the enzyme or microorganism; and
   (3) obtaining the (R)-1,3-butanediol produced in step (2);
[22] the method of [21], wherein the enzyme active substance in the above step (2) is any enzyme active substance selected from the group consisting of: the (R)-2,3-butanediol dehydrogenase of [1], the polypeptide of [6], a microorganism that produces either or both of them, and processed products thereof;
[23] the method of [21], wherein the enzyme active substance in the above step (1) is characterized as being a microorganism or processed products thereof, that produces (S) conformation-specific secondary alcohol dehydrogenase derived from *Candida parapucilosis*;
[24] the polynucleotide of [5], wherein the modification of (c) comprises conservative amino acid modification, such that the modified amino acid retains the amino acid side-chain properties of the original amino acid;
[25] the polynucleotide of [5], wherein the modified polypeptide of (c) has the amino acid sequence of SEQ ID NO: 15;
[26] the polynucleotide of [5], wherein the modification by addition of (c) results in a fusion protein;
[27] the polynucleotide of [5], wherein the number of amino acids modified by substitution, deletion, insertion and/or addition is less than 50;
[28] the polynucleotide of [5], wherein the number of amino acids modified by substitution, deletion, insertion and/or addition is less than 20;
[29] the polynucleotide of [5], wherein the number of amino acids modified by substitution, deletion, insertion and/or addition is less than 5;
[30] the polynucleotide of [5], wherein the polynucleotide encodes an enzyme possessing the properties recited in [1] and having at least 50% sequence identity with the polypeptide consisting of the amino acid sequence of SEQ ID NO: 2;
[31] the polynucleotide of [5], wherein the polynucleotide encodes an enzyme possessing the properties recited in [1] and having at least 70% sequence identity with the polypeptide consisting of the amino acid sequence of SEQ ID NO: 2;
[32] the polynucleotide of [5], wherein the polynucleotide encodes an enzyme possessing the properties recited in [1] and having at least 90% sequence identity with the polypeptide consisting of the amino acid sequence of SEQ ID NO: 2; and
[33] the polynucleotide of [5], wherein the polynucleotide encodes an enzyme possessing the properties recited in [1] and having at least 95% sequence identity with the polypeptide consisting of the amino acid sequence of SEQ ID NO: 2.

### BRIEF DESCRIPTION OF THE DRAWINGS

Figure 1 depicts a photograph demonstrating the pattern of SDS-PAGE. Lane 1 indicates the result of the molecular weight marker, and lane 2 that of the enzyme obtained in Example 1.

Figure 2 depicts a graph demonstrating the pH dependency of the (R)-1,3-butanediol oxidation activity of the enzyme obtained in Example 1.

Figure 3 depicts a graph demonstrating the pH dependency of the 4-hydroxy-2-butanone reduction activity of the enzyme obtained in Example 1.

Figure 4 depicts a graph demonstrating the temperature dependency of the (R)-1,3-butanediol oxidation activity of the enzyme obtained in Example 1.

Figure 5 depicts a graph demonstrating the temperature dependency of the 4-hydroxy-2-butanone reduction activity of the enzyme obtained in Example 1.

### DETAILED DESCRIPTION OF THE INVENTION

The (R)-2,3-butanediol dehydrogenase of the present invention is characterized by its ability to utilize NAD⁺ as a coenzyme, preferentially oxidizing the hydroxyl group of the (R) configuration of 2,3-butanediol, and producing optically active (R)-1,3-butanediol by reducing 4-hydroxy-2-butanone using NADH as a coenzyme. The (R)-2,3-butanediol dehydrogenase of the present invention denotes a dehydrogenase that has an activity to selectively oxidize the hydroxyl group of the (R) configuration of 2,3-butanediol, which not only oxidizes the hydroxyl group of the (2R,3R)-butanediol but also oxidizes the hydroxyl group with the (R) configuration of a meso-2,3-butanediol.

The enzyme of the present invention is dubbed (R)-2,3-butanediol dehydrogenase due to its potent enzyme activity on (R)-2,3-butanediol as indicated in the examples. Further, this enzyme also demonstrates a dehydrogenase action on (R)-1,3-butanediol.

According to the present invention, the oxidation activity on (R)-1,3-butanediol, reduction activity on 4-hydroxy-2-butanone, and reduction activity on 2,3-butanedion can be determined as follows.

### Assay method for the oxidation activity on (R)-1,3-butanediol:

A reaction solution containing 50 mM potassium phosphate buffer (pH 6.5), 2.5 mM NAD⁺, 20 mM (R)-1,3-butanediol, and the enzyme is reacted at 30°C, and the increase in absorbance at 340 nm due to the increase of NADH is measured. 1 U is defined as the amount of enzyme that catalyzes an increase of 1 µmol NADH in one minute. Further, the quantity of the protein is determined by the pigment bonding method using protein assay kit (Biorad).

### Assay method for the reduction activity on 4-hydroxy-2-butanone:

A reaction solution containing 50 mM potassium phosphate buffer (pH 6.5), 0.2 mM NADH, 20 mM 4-hydroxy-2-butanone, and the enzyme is reacted at 30°C, and the decrease in absorbance at 340 nm due to the decrease in NADH is measured. 1 U is defined as the amount of enzyme that catalyzes a decrease of 1 µmol NADH in one minute.

### Assay method for the reduction activity on 2,3-butanedione:

A reaction solution containing 50 mM potassium phosphate buffer (pH 6.5), 0.2 mM NADH, 20 mM 2,3-butanedione, and the enzyme is reacted at 30°C, and the decrease in absorbance at 340 nm due to a decrease in NADH is measured. 1 U is defined as the amount of enzyme that catalyses a decrease of 1 µmol NADH in one minute.

The (R)-2,3-butanediol dehydrogenase having the physicochemical properties as described above can be purified from a culture of, for example, yeast of the genus *Kluyveromyces.* As an example of a yeast belonging to the genus *Kluyveromyces, Kluyveromyces lactis* in particular has a superior ability to produce the (R)-2,3-butanediol dehydrogenase of the present invention. Examples of *Kluyveromyces lactis* that can be utilized to obtain the (R)-2,3-butanediol dehydrogenase of the present invention include the following strains, all of which can be obtained from the Institute for Fermentation in Osaka, Japan: IFO 1267, ATCC 8585, NRRL Y-1140, CBS 2359, IFO 1902, IFO 1903, and IFO 0433. Among these strains to obtain the (R)-2,3-butanediol dehydrogenase of the present invention, IFO 1267 is preferred.

The above microorganisms can be cultured in ordinary medium, such as YM medium, used for culturing fungi. After sufficient proliferation, the fungus is recovered and homogenated in a buffer solution containing a reducing agent, such as 2-mercaptoethanol or phenylmethane furfonylfluoride, or a protease inhibitor, to obtain the cell-free extract. Then, the enzyme can be purified by appropriately combining techniques, such as fractionation according to the solubility of the protein (such as precipitation using organic solvent or salting-out using ammonium sulfate); cation exchange; anion exchange; gel filtration; hydrophobic chromatography; and affinity chromatography using chelate, pigment, antibody, and so on. For example, the enzyme can be purified to a single band by electrophoresis after subjecting the extract to hydrophobic chromatography using phenyl-sepharose, anion exchange chromatography using MonoQ, hydrophobic chromatography using butyl-sepharose, adsorption chromatography using hydroxyapatite, and so on.

The (R)-2,3-butanediol dehydrogenase of the present invention derived from *Kluyveromyces lactis* is a polypeptide with physicochemical properties as follows:
(1) Action:
   the enzyme acts on (2R,3R)-2,3-butanediol, using NAD⁺ as a coenzyme, to produce (R)-acetoin. It reduces 2,3-butanedione, using NADH as a coenzyme, to produce (2R,3R)-2,3-butanediol;
(2) Substrate specificity:
   (a) the enzyme utilizes NAD⁺ as a coenzyme for the oxidation reaction and utilizes NADH as the coenzyme for the reduction reaction;
   (b) the enzyme reduces 4-hydroxy-2-butanone to produce (R)-1,3-butanediol; and
   (c) the enzyme preferentially oxidizes the hydroxyl group of the (R) configuration of racemic 1,3-butanediol to remain (S)-1,3-butanediol; and
   (3) Optimum pH:
   pH 6.0 for both the oxidation reaction and reduction reaction.

   Further, the (R)-2,3-butanediol dehydrogenase of the present invention is the dehydrogenase of claim 1 with additional physicochemical properties as follows:
   (4) Optimum temperature:
      37°C for both the oxidation reaction and reduction reaction; and
   (5) Molecular weight:
      the molecular weight determined by SDS-PAGE and gel filtration is 45,000 and 91,000, respectively.

The (R)-2,3-butanediol dehydrogenase derived from *Kluyveromyces lactis* is substantially unable to use β-nicotinamide adenine dinucleotide phosphate (hereinafter, abbreviated as NADP⁺) as a coenzyme in the oxidation reaction, or reduced β-nicotinamide adenine dinucleotide phosphate (hereinafter, abbreviated as NADPH) as a coenzyme in the reduction reaction. However, the enzymes with the above physicochemical properties of (1) to (3), and preferably with the additional physicochemical properties of (4) and (5), are encompassed in the enzymes of the present invention regardless of their ability to use NADP⁺ or NADPH.

The present invention relates to polynucleotides encoding (R)-2,3-butanediol dehydrogenases and homologues thereof.

As used herein, an "isolated polynucleotide" is a polynucleotide the structure of which is not identical to that of any naturally occurring polynucleotide or to that of any fragment of a naturally occurring genomic polynucleotide spanning more than three separate genes. The term therefore includes, for example, (a) a DNA which has the sequence of part of a naturally occurring genomic DNA molecule in the genome of the organism in which it naturally occurs; (b) a polynucleotide incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion polypeptide. Specifically excluded from this definition are polynucleotides of DNA molecules present in mixtures of different (i) DNA molecules, (ii) transfected cells, or (iii) cell clones; e.g., as these occur in a DNA library such as a cDNA or genomic DNA library.

Accordingly, in one aspect, the invention provides an isolated polynucleotide that encodes a polypeptide described herein or a fragment thereof. Preferably, the isolated polypeptide includes a nucleotide sequence that is at least 60% identical to the nucleotide sequence shown in SEQ ID NO: 1. More preferably, the isolated nucleic acid molecule is at least 65%, 70%, 75%, 80%, 85%, 90%, 91%, 92%, 93%, 94%, 95%, 96%, 97%, 98%, 99%, or more, identical to the nucleotide sequence shown in SEQ ID NO: 1. In the case of an isolated polynucleotide which is longer than or equivalent in length to the reference sequence, *e.g.*, SEQ ID NO: 1, the comparison is made with the full length of the reference sequence. Where the isolated polynucleotide is shorter than the reference sequence, *e.g.*, shorter than SEQ ID NO: 1, the comparison is made to segment of the reference sequence of the same length (excluding any loop required by the homology calculation).

According to the invention, the polynucleotides can be naturally occurring polynucleotides, such as DNAs or RNAs, or also artificial molecules including artificial nucleotide derivatives. Further, the polynucleotide of the present invention can be also chimeric molecules of DNA and RNA. There is no restriction on length of the polynucleotide of the present invention, but it preferably comprises at least 15 nucleotides.

The polynucleotide encoding the (R)-2,3-butanediol dehydrogenase of the present invention comprises, for example, the nucleotide sequence of SEQ ID NO: 1. The nucleotide sequence of SEQ ID NO: 1 encodes a polypeptide comprising the amino acid sequence of SEQ ID NO: 2. The polypeptide comprising this amino acid sequence constitutes preferred embodiments of the (R)-2,3-butanediol dehydrogenase of the present invention.

Homologues of a polynucleotide encoding the (R)-2,3-butanediol dehydrogenase of the present invention include polynucleotides that both encode a polypeptide having the above-mentioned physicochemical properties (1) to (3) and comprise the amino acid sequence of SEQ ID NO: 2 in which one or more amino acids are deleted, substituted, inserted, and/or added. One skilled in the art can properly introduce substitution, deletion, insertion, and/or addition mutation into the polynucleotide of SEQ ID NO: 1 by site-specific mutagenesis (Nucleic Acid Res. 10, pp. 6487 (1982), Methods in Enzymol. 100, pp. 448 (1983); Molecular Cloning 2nd Ed., Cold Spring Harbor Laboratory Press (1989), PCR: A Practical Approach, IRL Press, pp. 200 (1991)).

In addition, a homologue of a polynucleotide of the present invention includes polynucleotides hybridizing under stringent conditions to the polynucleotides that comprise the nucleotide sequence of SEQ ID NO: 1, as well as those encoding a polypeptide having the above-mentioned physicochemical properties (1) to (3). The phrase "polynucleotide hybridizing under stringent conditions" refers to a polynucleotide hybridizing to a probe nucleotide that has one or more segments of at least 20 consecutive nucleotides, preferably at least 30 consecutive nucleotides, for example, 40, 60, or 100 consecutive nucleotides, arbitrarily selected from the sequence of SEQ ID NO: 1, using, for example, ECL Direct Nucleic Acid Labeling and Detection System (Amersham-Pharmacia Biotech) under conditions recommended in the attached manual (washing with the primary wash buffer containing 0.5x SSC at 42°C). Also included in the invention is a polynucleotide that hybridizes under high stringency conditions to the nucleotide sequence of SEQ ID NO: 1 or a segment thereof as described herein. "High stringency conditions" refers to hybridization in 6x SSC at about 45°C, followed by one or more washes in 0.2x SSC, 0.1% SDS at 65°C.

Furthermore, a homologue of a polynucleotide of the present invention includes a polynucleotide encoding a polypeptide exhibiting an identity of at least 80%, preferably at least 85% or 90%, more preferably 95% or more to the amino acid sequence of SEQ ID NO: 2. As used herein, "percent identity" of two amino acid sequences or of two nucleic acids is determined using the algorithm of Karlin and Altschul (Proc. Natl. Acad. Sci. USA 87: 2264-2268, 1990) modified as in Karlin and Altschul (Proc. Natl. Acad. Sci. USA 90:5873-5877, 1993). Such an algorithm is incorporated into the NBLAST and XBLAST programs of Altschul et al. (J. Mol. Biol. 215:403-410, 1990). BLAST nucleotide searches are performed with the NBLAST program, score = 100, wordlength = 12. Homology search of protein can readily be performed, for example, in DNA Databank of JAPAN (DDBJ), by using the FASTA program, BLAST program, etc. BLAST protein searches are performed with the XBLAST program, score = 50, wordlength = 3. Where gaps exist between two sequences, Gapped BLAST is utilized as described in Altsuchl et al. (Nucleic Acids Res. 25: 3389-3402, 1997). When utilizing BLAST and Gapped BLAST programs, the default parameters of the respective programs (e.g., XBLAST and NBLAST) are used. Homology search of protein can be readily performed, for example, via Internet, for example, in databases related to amino acid sequences of protein, such as SWISS-PROT, PIR, and such; databases related to DNAs, such as DDBJ, EMBL, or GenBank, and such; databases related to deduced amino acid sequences based on DNA sequences; and such using programs, such as BLAST, FASTA, etc.

As a result of a homology search by the BLAST program focusing on SWISS-PROT using the amino acid sequence of SEQ ID NO: 2, YAGO of *Saccharomyces cerevisiae* demonstrated the highest homology among known polypeptides. YAGO is a hypothetical zinc-type alcohol dehydrogenase-like protein that was predicted as results of genome analysis, and the existence, function, physicochemical properties, and so on of the protein remains completely unknown. The amino acid sequence homology to YAGO was 60% as "Identity" and 73% as "Positive". 80% or more homology in the present invention represents a homology value determined as "Positive" by the BLAST program.

The present invention relates to a polypeptide comprising the amino acid sequence of SEQ ID NO: 2. The present invention also includes homologues of a polypeptide comprising the amino acid sequence of SEQ ID NO: 2.

The term "substantially pure" as used herein in reference to a given polypeptide means that the polypeptide is substantially free from other biological macromolecules. The substantially pure polypeptide is at least 75% (e.g., at least 80, 85, 95, or 99%) pure by dry weight. Purity can be measured by any appropriate standard method, for example by column chromatography, polyacrylamide gel electrophoresis, or HPLC analysis.

Homologues of the (R)-2,3-butanediol dehydrogenase of the present invention may comprise the amino acid sequence of SEQ ID NO: 2 in which one or more amino acids are deleted, substituted, inserted, and/or added, wherein said dehydrogenase is functionally equivalent to the polypeptide consisting of the amino acids of SEQ ID NO: 2. According to the present invention, "polypeptides functionally equivalent to the polypeptide consisting of the amino acid sequence of SEQ ID NO: 2" are those polypeptides having the above-mentioned physicochemical properties (1) to (3). One skilled in the art can readily obtain a polynucleotide encoding such homologues of the (R)-2,3-butanediol dehydrogenase by properly introducing substitution, deletion, insertion, and/or addition mutation into the polynucleotide of SEQ ID NO: 1 by site-specific mutagenesis (Nucleic Acid Res. 10, pp. 6487 (1982), Methods in Enzymol. 100, pp. 448 (1983); Molecular Cloning 2nd Ed., Cold Spring Harbor Laboratory Press (1989), PCR: A Practical Approach IRL Press pp. 200 (1991)) or the like. A homologue of the (R)-2,3-butanediol dehydrogenase of SEQ ID NO: 2 can be obtained by introducing into a host polynucleotide encoding the homologue of the (R)-2,3-butanediol dehydrogenase and expressing it in the host.

The number of amino acids that are mutated is not particularly restricted, as long as the (R)-2,3-butanediol dehydrogenase activity is maintained. Normally, it is within 50 amino acids, preferably within 30 amino acids, more preferably within 10 amino acids, and even more preferably within 3 amino acids. The site of mutation may be any site, as long as the (R)-2,3-butanediol dehydrogenase activity is maintained.

An amino acid substitution is preferably mutated into different amino acid(s) in which the properties of the amino acid side-chain are conserved. A "conservative amino acid substitution" is a replacement of one amino acid residue belonging to one of the following groups having a chemically similar side chain with another amino acid in the same group. Groups of amino acid residues having similar side chains have been defined in the art. These groups include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Furthermore, homologues of the (R)-2,3-butanediol dehydrogenase of the present invention includes polypeptides exhibiting percent identity of at least 80%, preferably at least 85% or 90%, more preferably 95% or more to the amino acid sequence of SEQ ID NO: 2. Homology search of protein can readily be performed, for example, via the Internet, for example, in databases related to amino acid sequences of protein, such as SWISS-PROT, PIR, and such; databases related to DNA sequences, such as DDBJ, EMBL, GenBank, and such; databases related to deduced amino acid sequences based on DNA sequences; and such by using programs, such as BLAST, FASTA, etc.

The polynucleotide encoding the (R)-2,3-butanediol dehydrogenase of the present invention may be isolated, for example, using the following procedure.

The DNA of this invention can be obtained by PCR by designing primers for PCR based on the nucleotide sequence of SEQ ID NO: 1 and using chromosomal DNA or cDNA library of the enzyme-producing strain as a template.

Furthermore, using the obtained DNA fragment as a probe, the polynucleotide of this invention can be obtained by colony or plaque hybridization using a library obtained by transforming *E. coli* with a phage or plasmid into which restriction enzyme digestion products of the chromosomal DNA of the enzyme-producing strain are introduced, or a cDNA library.

In addition, the polynucleotide of the present invention can be obtained by analyzing the nucleotide sequence of the DNA fragment obtained by PCR, by designing PCR primers, based on the sequence already obtained, to extend the DNA outward, by digesting, with an appropriate restriction enzyme(s), the chromosomal DNA of the strain producing the enzyme of interest and then, by performing inverse PCR (Genetics 120, 621-623 (1988)) using self-ligated circular DNA as a template; or alternatively by the RACE method (Rapid Amplification of cDNA End; "Experimental manual for PCR" pp. 25-33, HBJ Press).

The polynucleotides of the present invention include not only genomic DNA and cDNA cloned by the above-mentioned methods but also chemically synthesized polynucleotide.

The thus-isolated polynucleotide encoding the (R)-2,3-butanediol dehydrogenase of the present invention can be inserted into a known expression vector to provide a (R)-2,3-butanediol dehydrogenase-expressing vector.

Further, by culturing cells transformed with the expression vector, the (R)-2,3-butanediol dehydrogenase of the present invention can be obtained from the transformed cells.

In the present invention, there is no restriction on the microorganism to be transformed to express (R)-2,3-butanediol dehydrogenase, so long as the organism is capable of being transformed with the vector containing the polynucleotide encoding the polypeptide with the activity of (R)-2,3-butanediol dehydrogenase and capable of expressing the active (R)-2,3-butanediol dehydrogenase. Available microorganisms are those for which host-vector systems are available and include, for example:
bacteria, such as the genus *Escherichia,* the genus *Bacillus,* the genus *Pseudomonas,* the genus *Serratia,* the genus *Brevibacterium*, the genus *Corynebacterium,* the genus *Streptococcus,* and the genus *Lactobacillus*;
actinomycetes, such as the genus *Rhodococcus* and the genus *Streptomyces*;
yeasts, such as the genus *Saccharomyces*, the genus *Kluyveromyces,* the genus *Schizosaccharomyces,* the genus *Zygosaccharomyces,* the genus *Yarrowia,* the genus *Trichosporon,* the genus *Rhodosporidium,* the genus *Pichia,* and the genus *Candida;* and
fungi, such as the genus *Neurospora,* the genus *Aspergillus,* the genus *Cephalosporium,* and the genus *Trichoderma*; etc.

Procedures for the preparation of a transformant and construction of a recombinant vector suitable for a host can be carried out by employing techniques that are commonly used in the fields of molecular biology, bioengineering, and genetic engineering (for example, see Sambrook et al., "Molecular Cloning", Cold Spring Harbor Laboratories). In order to express, in a microorganism, the gene encoding the (R)-2,3-butanediol dehydrogenase of the present invention whose electron donor is NADH, it is necessary to introduce the polynucleotide into a plasmid vector or phage vector that is stable in the microorganism and to let the genetic information be transcribed and translated.

To do so, a promoter, a unit for regulating transcription and translation, is preferably placed upstream of the 5' end of the polynucleotide of the present invention, and preferably a terminator is placed downstream of the 3' end of the polynucleotide. The promoter and the terminator should be functional in the microorganism to be utilized as a host. Available vectors, promoters, and terminators for the above-mentioned various microorganisms are described in detail in "Fundamental Course in Microbiology (8): Genetic Engineering", Kyoritsu Shuppan, specifically for yeasts, in "Adv. Biochem. Eng. 43, 75-102(1990)" and "Yeast 8, 423-488 (1992) ".

For example, for the genus *Escherichia,* in particular, for *Escherichia coli,* available plasmids include pBR series and pUC series plasmids; available promoters include those derived from lac (derived from β-galactosidase gene), trp (derived from the tryptophan operon), tac and trc (which are chimeras of lac and trp), P_{L} and P_{R} of λphage, etc. Available terminators include those derived from trpA, phages, rrnB ribosomal RNA, etc. Among these plasmids, vector pSE420D (described in JP-A 2000-189170) wherein a part of the multi cloning site of commercially available pSE420 (Invitrogen) is modified can be preferably used.

For the genus *Bacillus,* available vectors are pUB110 series and pC194 series plasmids; the vectors can be integrated into host chromosome. Available promoters and terminators are derived from apr (alkaline protease) , npr (neutral protease) , amy (α-amylase), etc.

For the genus *Pseudomonas,* there are host-vector systems developed for *Pseudomonas putida* and *Pseudomonas cepacia.* A broad-host-range vector, pKT240, (containing RSF1010-derived genes required for autonomous replication) based on TOL plasmid, which is involved in decomposition of toluene compounds, is available; a promoter and a terminator derived from the lipase gene (JP-A No. Hei 5-284973) are available.

For the genus *Brevibacterium,* in particular, for *Brevibacterium lactofermentum,* available plasmid vectors include pAJ43 (Gene 39, 281 (1985)). Promoters and terminators used for *Escherichia coli* can be utilized without any modification for *Brevibacterium.*

For the genus *Corynebacterium,* in particular, for *Corynebacterium glutamicum,* plasmid vectors such as pCS11 (JP-A Sho 57-183799) and pCB101 (Mol. Gen. Genet. 196, 175(1984)) are available.

For the genus *Streptococcus,* plasmid vectors such as pHV1301 (FEMS Microbiol. Lett. 26, 239 (1985)) and pGK1 (Appl. Environ. Microbiol. 50, 94 (1985)) can be used.

For the genus *Lactobacillus,* plasmid vectors such as pAMβ1 (J. Bacteriol. 137, 614 (1979)), which was developed for the genus *Streptococcus,* can be utilized; and promoters that are used for *Escherichia coli* are also usable.

For the genus *Rhodococcus,* plasmid vectors isolated from *Rhodococcus rhodochrous* are available (J. Gen. Microbiol. 138, 1003 (1992)).

For the genus *Streptomyces,* plasmids can be constructed in accordance with the method as described in "Genetic Manipulation of *Streptomyces:* A Laboratory Manual" (Cold Spring Harbor Laboratories (1985)) by Hopwood et al. In particular, for *Streptomyces lividans,* pIJ486 (Mol. Gen. Genet. 203, 468-478, 1986), pKC1064 (Gene 103, 97-99 (1991)), and pUWL-KS (Gene 165, 149-150 (1995)) are usable. The same plasmids can also be utilized for *Streptomyces virginiae* (Actinomycetol. 11, 46-53 (1997)).

For the genus *Saccharomyces*, in particular, for *Saccharomyces cerevisiae,* YRp series, YEp series, YCp series, and YIp series plasmids are available; integration vectors (refer EP 537456, etc.), which are integrated into chromosome via homologous recombination with multicopy-ribosomal genes, allow to introduce a gene of interest in multicopy and the gene incorporated is stably maintained in the microorganism; and thus, these types of vectors are highly useful. Available promoters and terminators are derived from genes encoding ADH (alcohol dehydrogenase), GAPDH (glyceraldehyde-3-phosphate dehydrogenase), PHO (acid phosphatase), GAL (β-galactosidase), PGK (phosphoglycerate kinase), ENO (enolase), etc.

For the genus *Kluyveromyces,* in particular, for *Kluyveromyces lactis,* available plasmids are those such as 2- m plasmids derived from *Saccharomyces cerevisiae,* pKD1 series plasmids (J. Bacteriol. 145, 382-390(1981)), plasmids derived from pGKl1 and involved in the killer activity, KARS (*Kluyveromyces* autonomous replication sequence) plasmids, and plasmids (refer EP 537456, etc.) capable of being integrated into chromosome via homologous recombination with the ribosomal DNA. Promoters and terminators derived from ADH, PGK, and the like are available.

For the genus *Schizosaccharomyces,* it is possible to use plasmid vectors comprising ARS (autonomous replication sequence) derived from *Schizosaccharomyces pombe* and auxotrophy-complementing selectable markers derived from *Saccharomyces cerevisiae* (Mol. Cell. Biol. 6, 80 (1986)). Promoters such as ADH promoter derived from *Schizosaccharomyces pombe* are usable (EMBO J. 6, 729 (1987)). In particular, pAUR224 is commercially available from TaKaRa Shuzo Co., Ltd.

For the genus *Zygosaccharomyces*, plasmids originating from those such as pSB3 (Nucleic Acids Res. 13, 4267 (1985)) derived from *Zygosaccharomyces rouxii* are available; it is possible to use promoters such as PHO5 promoter derived from *Saccharomyces cerevisiae* and GAP-Zr (Glyceraldehyde-3-phosphate dehydrogenase) promoter (Agri. Biol. Chem. 54, 2521 (1990)) derived from *Zygosaccharomyces rouxii.*

For the genus *Pichia,* host vector systems are developed for *Pichia angusta* (previously called *Hansenula polymorpha).* Although autonomous replication sequences (HARS1 and HARS2) derived from *Pichia angusta* are available as vectors, they are rather unstable. Therefore, multicopy integration to chromosome is effective for them (Yeast 7, 431-443 (1991)). Further, promoters, such as AOX (alcohol oxidase) and FDH (formate dehydrogenase) induced by methanol are available. Furthermore, host-vector systems originating from autonomous replication sequences (PARS1, PARS2) derived from *Pichia* have been developed (Mol. Cell. Biol. 5, 3376 (1985)), and it is possible to employ a highly efficient promoter such as methanol-inducible AOX promoter, which is available for high-cell-density-culture (Nucleic Acids Res. 15, 3859 (1987)).

For the genus *Candida,* host-vector systems have been developed for *Candida maltosa, Candida albicans, Candida tropicalis, Candida utilis,* etc. An autonomous replication sequence originating from *Candida maltosa* has been cloned (Agri. Biol. Chem. 51, 51, 1587 (1987)), and a vector using the sequence has been developed for *Candida maltosa.* Further, a chromosome-integration vector with a highly efficient promoter unit has been developed for *Candida utilis* (JP-A Hei 08-173170).

For the genus *Aspergillus, Aspergillus niger* and *Aspergillus oryzae* have intensively been studied among fungi, and thus plasmid vectors and chromosome-integration vectors are known in the art and available, as well are promoters derived from an extracellular protease gene and amylase gene (Trends in Biotechnology 7, 283-287 (1989)).

For the genus *Trichoderma,* host-vector systems have been developed for *Trichoderma reesei,* and promoters such as those derived from extracellular cellulase genes are available (Biotechnology 7, 596-603(1989)).

There are various host-vector systems developed for plants and animals other than microorganisms; in particular, the systems include those of insect such as silkworm (Nature 315, 592-594(1985)), and plants such as rapeseed, maize, potato, etc. These systems are preferably employed to express a large amount of foreign polypeptide.

Microorganisms that possess the ability to produce (R)-2,3-butanediol dehydrogenase of this invention include all strains, mutants, variants, and transformants that have the ability to produce (R)-2,3-butanediol dehydrogenase and that belong to the genus *Kluyveromyces,* transformants being constructed by genetic engineering to confer the ability to produce the enzyme of this invention. The transformants can be used in the form of the culture, cells separated from the culture medium by filtration, centrifugation or the like, or cells resuspended in buffer, water, or the like after they are separated by centrifugation and washed. The separated transformants can be used in a state as they are recovered, as their disrupts, as treated with acetone or toluene, or as lyophilizate. When the enzyme is extracellularly produced, the culture medium of the transformants can also be used after it is separated from the transformants by the usual methods.

The present invention relates to methods for producing optically active alcohols by reducing ketones or methods for producing ketones by oxidizing alcohols utilizing an enzyme active substance having the above (R)-2,3-butanediol dehydrogenase activity. According to the present invention, the term "enzyme active substance having (R)-2,3-butanediol dehydrogenase activity" refers to any substance selected from the group consisting of the above-mentioned (R)-2,3-butanediol dehydrogenases, recombinant polypeptides, microorganisms that produces them, and treated or processed products thereof.

Examples of processed products of the microorganism of the present invention include: microorganisms with a cell membrane permeability modified by a treatment with surfactants or organic solvents, such as toluene; cell-free extracts obtained by homogenizing microorganisms with glass beads or enzyme treatment; and partial purified products thereof. Moreover, enzyme proteins coupled, adsorbed, or embedded on carriers are examples of processed products of the (R)-2,3-butanediol dehydrogenase or recombinant polypeptide thereof. Not only insoluble carriers but also water-soluble carriers may be used as the carrier. Methods for obtaining processed products of the enzyme protein using carriers are known in the art.

The present invention further relates to methods for producing optically active alcohols comprising the steps of: contacting the enzyme active substance with a ketone in the presence of NADH; and obtaining the optically active alcohol produced by the reduction of the ketone. Further, the present invention relates to methods for producing optically active alcohols comprising the steps of: contacting the enzyme active substance with a racemic alcohol, in the presence of NAD⁺, to oxidize one of the optical isomers, and obtain the remaining optically active alcohol.

Examples of ketones that are preferably used in the method for producing optically active alcohols according to the present invention include 2,3-butanedione, 2,3-pentanedione, and 4-hydroxy-2-butanone. For example, (R)-1,3-butanediol can be synthesized in the case of using 4-hydroxy-2-butanone as the substrate.

In addition, (R)-1,3-butanediol is selectively oxidized to obtain the remaining optically active alcohol, (S)-1,3-butanediol, by contacting the enzyme active substance with racemic 1,3-butanediol as the starting material.

Moreover, the present invention relates to methods for producing desired optically active alcohols from racemic alcohols using both microorganisms that produce alcohol dehydrogenases with the opposite stereoselectivity to that of the enzyme of the present invention and those producing the (R)-2,3-butanediol dehydrogenase of the present invention. More specifically, the present invention provides methods for producing (R)-1,3-butanediol comprising the steps of:
(1)synthesizing (R)-1,3-butanediol and 4-hydroxy-2-butanone by contacting any enzyme active substance selected from the group consisting of: an enzyme that preferentially oxidizes (S)-1,3-butanediol to produce 4-hydroxy-2-butanone; a microorganism that produces the enzyme; and processed products thereof with racemic 1,3-butanediol;
(2)reducing 4-hydroxy-2-butanone to produce (R)-1,3-butanediol by contacting any enzyme active substance selected from the group consisting of: an enzyme that reduces the 4-hydroxy-2-butanone to produce (R)-1,3-butanediol; a microorganism that produces the enzyme, and processed products thereof with the 4-hydroxy-2-butanone produced in step (1); and
(3)obtaining the (R)-1,3-butanediol that is produced in step (2).

Steps (1) and (2) above can be carried out either sequentially or simultaneously.

Examples of microorganisms having the activity to selectively oxidize (S)-1,3-butanediol in racemic 1,3-butanediol include, for example, microorganisms known to produce (R)-1,3-butanediol from racemic 1,3-butanediol described in JP-A Hei 02-195897, JP-A Hei 03-187399, JP-A Hei 04-152895, and JP-A Hei 03-228693.

On the other hand, examples of enzymes having the activity to selectively oxidize (S)-1,3-butanediol of racemic 1,3-butanediol include (S)-specific secondary alcohol dehydrogenases exemplified by (S)-specific secondary alcohol dehydrogenase produced by *Candida parapucilosis* (JP-A Hei 07-231785), the (S)-specific secondary alcohol dehydrogenase produced by *Geotrichum candidum* (WO98/17788), and so on. Among these enzymes, preferable examples are those obtained by highly expressing a gene encoding the (S)-specific secondary alcohol dehydrogenase produced by *Candida parapucilosis* in *Escherichia coli* (JP-A Hei 07-231785).

Moreover, the present invention relates to methods for producing ketones comprising a step of contacting the above enzyme active substance with an alcohol, in the presence of NAD⁺, to obtain the ketone produced by the oxidation of the alcohol.

Examples of alcohols in the methods for producing ketones of the present invention include (2R,3R)-2,3-butanediol and meso-2,3-butanediol. (R)-acetoin and (S)-acetoin can be respectively synthesized from these compounds as the substrate. Furthermore, 4-hydroxy-2-butanone can be synthesized from (R)-1,3-butanediol.

The desired enzyme reaction can be carried out by contacting enzyme molecules, processed products thereof, culture containing the enzyme molecules, or transformants of microorganisms that produce the enzyme with a reaction solution containing a substrate and coenzyme. It should be noted that the form of contact between the enzyme and reaction solution is not limited to these specific examples.

Further, an enzyme reaction for regenerating the NAD⁺, produced from NADH during the reduction reaction, to NADH can be combined with the method for producing optically active alcohol of the present invention. The enzyme reaction for regenerating NADH can be carried out, for example, utilizing the ability of microorganisms to reduce NAD⁺ (the glycolysis pathway, the C1 compound assimilation pathway of a methylotroph, and so on). Such an ability to regenerate NAD⁺ can be reinforced by adding glucose, ethanol, formic acid, and such to the reaction system.

Alternatively, microorganisms generating NADH from NAD⁺, or processed products or enzyme thereof can be added to the reaction system to regenerate NADH. For example, regeneration of NADH can be carried out using microorganisms possessing glucose dehydrogenase, formate dehydrogenase, alcohol dehydrogenase, amino acid dehydrogenase, organic acid dehydrogenase (such as malate dehydrogenase), or the like, or processed products or partial purified or purified enzymes thereof. Reactants necessary for NADH regeneration reaction can be added to the reaction system for producing alcohol of the present invention as they are, or as their immobilized products. Alternatively, the reactants can be also contacted with the reaction system through a membrane transparent to NADH.

When viable cells of the microorganism transformed with a recombinant vector including a polynucleotide of this invention are used to produce the above alcohols, an additional reaction system for NADH regeneration can be sometimes omitted. Specifically, by using microorganisms that have a high activity of NADH regeneration, efficient reduction reaction using transformants can be performed without adding enzymes for NADH regeneration.

Moreover, by introducing, into a host, a gene for glucose dehydrogenase, formate dehydrogenase, alcohol dehydrogenase, amino acid dehydrogenase, organic acid dehydrogenase (for example, malate dehydrogenase, etc.), and such, which are available for NADH regeneration, simultaneously with a polynucleotide encoding the NADH-dependent (R)-2,3-butanediol dehydrogenase of the present invention, more efficient expression of the NADH regeneration enzyme and the NAD⁺-dependent (R)-2,3-butanediol dehydrogenase, and reduction reaction can be performed. Examples of methods for introducing these two or more genes into a host include: a method for transforming the host with multiple recombinant vectors obtained by separately introducing, to avoid incompatibility, the genes into multiple vectors whose replication origins are different; a method in which both genes are introduced into a single vector; and a method for introducing one or both genes into chromosomes.

When multiple genes are introduced into a single vector, each gene can be ligated to the region involved in the regulation of expression, such as promoter or terminator region. Multiple genes can be also expressed as operons including multiple cistrons, such as the lactose operon.

Further, an enzyme reaction for regenerating NADH, produced from NAD⁺ during the oxidization reaction, to NAD⁺ can be combined with the method for producing optically active alcohols or ketones of the present invention. The method for producing optically active alcohols of the present invention comprises the step of oxidizing one of the optical isomers that compose a racemate to remain the other optical isomer.

Regeneration of NADH, produced during the oxidization reaction from NAD⁺, to NAD⁺ can be generally carried out utilizing the NADH oxidation activity of a microorganism. More specifically, the reaction preferably utilizes the action of enzymes that compose the electron transfer system of cells, such as NADH dehydrogenase, NADH oxidase, and diaphorase (J. Am. Chem. Soc. 107, 6999-7008 (1985)). When *Escherichia coli* and such are used as the host, a microorganism with high NAD⁺ regeneration activity can be prepared by culturing the microorganism having a gene encoding the (R)-2,3-butanediol dehydrogenase of the present invention and maintaining a low dissolved oxygen concentration (JP-A 2000-197485).

In addition, NAD⁺ regeneration can be carried out more efficiently by introducing a gene encoding NADH dehydrogenase, NADH oxidase, diaphorase, and so on into a host along with a gene encoding the (R)-2,3-butanediol dehydrogenase of the present invention, in the same manner as the method described for NADH.

A reaction using the enzyme of the present invention can be carried out in water; an organic solvent that is insoluble in water, for example, ethyl acetate, butyl acetate, toluene, chloroform, n-hexane, and so on; a biphasic system with an aqueous medium; or a mixed system with organic solvent that is soluble in water, for example, methanol, ethanol, isopropyl alcohol, acetonitrile, acetone, dimethyl sulfoxide, and so on. The reaction of the present invention can be also carried out using immobilized enzymes, film reactors, and so on.

The reaction of the present invention can be carried out at a reaction temperatures ranging from 4 to 60°C, preferably 15 to 30°C, at a pH of 3 to 11, preferably 6 to 9.5, and with a substrate concentration ranging from 0.01 to 90%, preferably 0.1 to 30%. If necessary, 0.001 mM to 100 mM or preferably 0.01 to 10 mM coenzyme, NAD⁺ or NADH, can be added to the reaction system. Although the substrate can be added at once at the start of reaction, it is preferably added continually or discontinuously so as not to make the concentration of the substrate in the reaction mixture too high.

For regenerating NADH, for example, glucose in case of using glucose dehydrogenase, formic acid in case of using formate dehydrogenase, or ethanol or isopropanol in case of using alcohol dehydrogenase is added to the reaction system. These compounds can be added at a molar ratio of 0.1-20 times, preferably in 1 to 5 times as much as the substrate ketone. On the other hand, enzymes for regenerating NADH, such as glucose dehydrogenase, formic acid dehydrogenase, and alcohol dehydrogenase, can be added 0.1 to 100 times, preferably 0.5 to 20 times as much as the NADH-dependent carbonyl dehydrogenase of the present invention by the enzyme activity.

An efficient enzyme reaction is enabled by adapting the amount of (R)-2,3-butanediol dehydrogenase of the present invention in the 4-hydroxy-2-butanone reducing reaction to, for example, 1 mU/ml to 100 U/ml (as the 4-hydroxy-2-butanone reduction activity), and preferably 100 mU/ml or more, relative to the above substrate concentrations. Further, when using cell bodies of microorganism as the enzyme active substance, the amount of microorganism used relative to the substrate is preferably an amount equivalent to 0.01 to 5.0 wt% by the weight of dried cell bodies of the microorganism. The enzymes or enzyme active substances, such as cell bodies of the microorganism, can be brought into contact with the substrate by dissolving or dispersing it into the reaction solution. Alternatively, enzyme active substances immobilized by methods, such as chemical bonding or inclusion, can be also used. Moreover, the reaction can be also carried out in a state wherein the substrate solution and enzyme active substance are separated by a porous membrane, that allows the permeation of substrate but restricts the permeation of enzyme molecules and microorganisms.

Purification of alcohols produced by the reduction of ketones of the present invention can be carried out by suitably combining separation, solvent extraction, distillation, and so on with centrifugation, membrane treatment, and such of microorganisms and polypeptides.

For example, (R)-1,3-butanediol is extracted into the solvent layer after removing cell bodies of the microorganism by centrifugation of the reaction solution containing the microorganism, and adding solvent, such as ethyl acetate, to the filtrate. Then, the solvent phase is separated and distilled to purify the (R)-1,3-butanediol to a high purity.

The present invention is specifically illustrated below with reference to Examples, but is not construed as being limited thereto.

The present invention provides an NAD⁺-dependent (R)-2,3-butanediol dehydrogenase that is useful for the production of optically active alcohol and so on. The use of the enzyme enables efficient methods for producing (R)-1,3-butanediol of high optical purity from 4-hydroxy-2-butanone, methods for producing (S)-1,3-butanediol of high optical purity from racemic 1,3-butanediol, and efficient methods for producing 4-hydroxy-2-butanone from (R)-1,3-butanediol.

Any patents, patent applications, and publications cited herein are incorporated by reference.

Herein, "%" for concentration denotes weight per volume percent unless otherwise specified.

### [Example 1] Purification of the (R)-2,3-butanediol dehydrogenase

*Kluyveromyces lactis* strain IFO 1267 was cultured in 1.2 liters of YM medium (glucose 20 g/L, yeast extract 3 g/L, malt extract 3 g/L, peptone 5 g/L, pH 6.0) and the cell bodies were prepared by centrifugation. The resulting wet cell bodies were suspended in 50 mM potassium phosphate buffer (pH 8.0), containing 0.02% 2-mercaptoethanol and 2 mM phenylmethane sulfonylfluoride (PMSF), and after homogenizing with Bead Beater (Biospec), the residue of the cell bodies were removed by centrifugation to obtain a cell-free extract. Protamine sulfate was then added to the cell-free extract followed by centrifugation to obtain enucleated supernatant. Ammonium sulfate was added to the supernatant to 30% saturation, and the resulting mixture was added to phenyl-sepharose HP column (2.6 cm x 10 cm) equilibrated with standard buffer solution (10 mM Tris-HCl buffer (pH 8.5), 0.01% 2-mercaptoethanol, and 10% glycerol) containing 30% ammonium sulfate. Then, gradient elution with ammonium sulfate concentrations of 30% to 0% was carried out. Three peaks of NADH-dependent 4-hydroxy-2-butanone reduction activity were observed in the gradient eluted fractions. The peak that eluted first among these peaks was collected and concentrated by ultrafiltration.

After dialyzing the concentrated enzyme solution against the standard buffer solution, the solution was loaded on a MonoQ column (0.5 cm x 5 cm), equilibrated with the same buffer solution, followed by gradient elution with 0 to 0.5 M sodium chloride. The eluted fraction with 4-hydroxy-2-butanone reduction activity was collected and concentrated by ultrafiltration.

Ammonium sulfate was added to the concentrated enzyme solution to 40% saturation, and the solution was loaded on a butyl-sepharose column (0.75 cm x 2.5 cm), equilibrated with standard buffer solution containing 40% saturated ammonium sulfate, followed by gradient elution with 40% to 0% saturated ammonium sulfate. The eluted fraction with 4-hydroxy-2-butanone reduction activity was collected.

After dialyzing the fraction having the 4-hydroxy-2-butanone reduction activity with 5 mM potassium phosphate buffer solution (pH 8.0) containing 0.01% 2-mercaptoethanol and 10% glycerol, the fraction was loaded on a hydroxyapatite column (0.5 cm x 10 cm), equilibrated with the same buffer, followed by concentration gradient elution with 5 to 350 mM potassium phosphate buffer solution (pH 8.0).

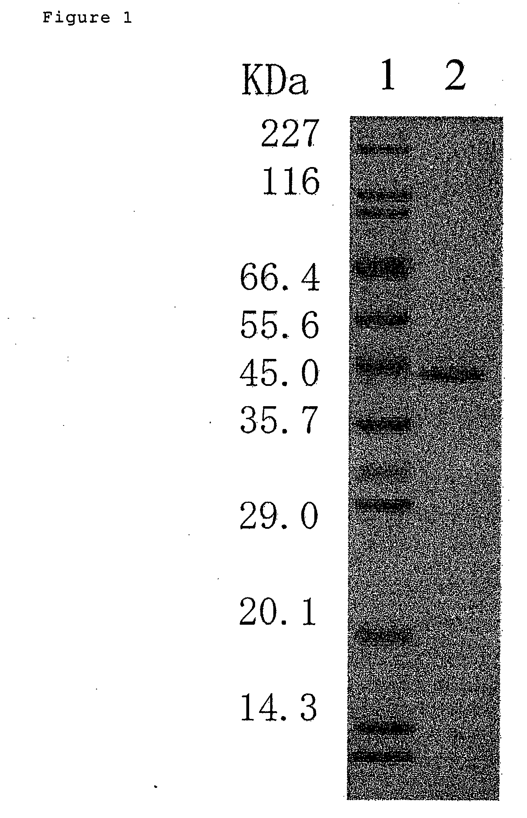

As a result of SDS-PAGE analysis, the fraction with 4-hydroxy-2-butanone reduction activity obtained from the hydroxyapatite column indicated a single band (Figure 1).

The specific activity of the purified enzyme was about 1.5 U/mg. A summary of the purification is shown in Table 1.

**Table 1**

| Step | Protein (mg) | Enzyme activity (U) | Specific activity (U/mg) |
|---|---|---|---|
| Cell-free extract | 3390 | 16.2 | 0.00478 |
| Protamine sulfate precipitate | 1480 | 12.5 | 0.00845 |
| Phenyl-sepharose | 50.8 | 2.08 | 0.0410 |
| MonoQ | 2.23 | 1.27 | 0.570 |
| Butyl-sepharose | 0.559 | 0.394 | 0.705 |
| Hydroxyapatite | 0.0325 | 0.0483 | 1.484 |

### [Example 2] Molecular weight measurement of the (R)-2,3-butanediol dehydrogenase

The molecular weight of the subunit of the enzyme obtained in Example 1 determined by SDS-PAGE was 45,000, whereas the molecular weight determined by a Superdex G200 gel filtration column was 91,000.

### [Example 3] Optimal pH of the (R)-2,3-butanediol dehydrogenase

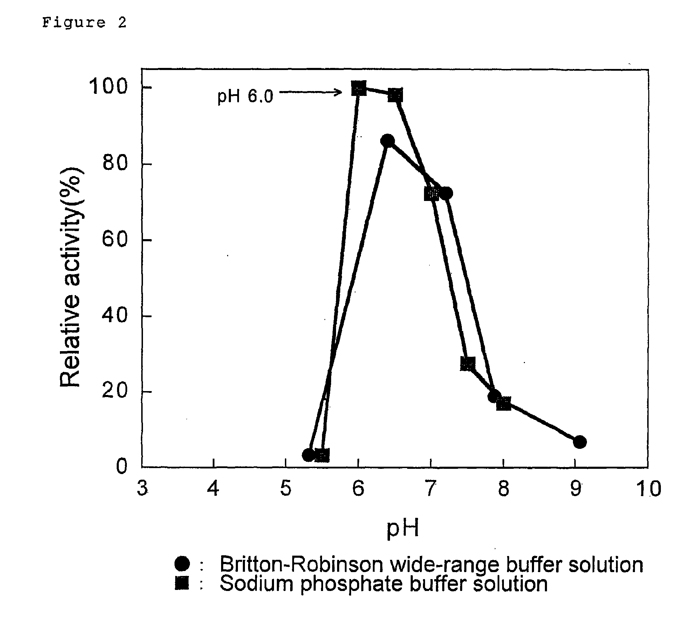

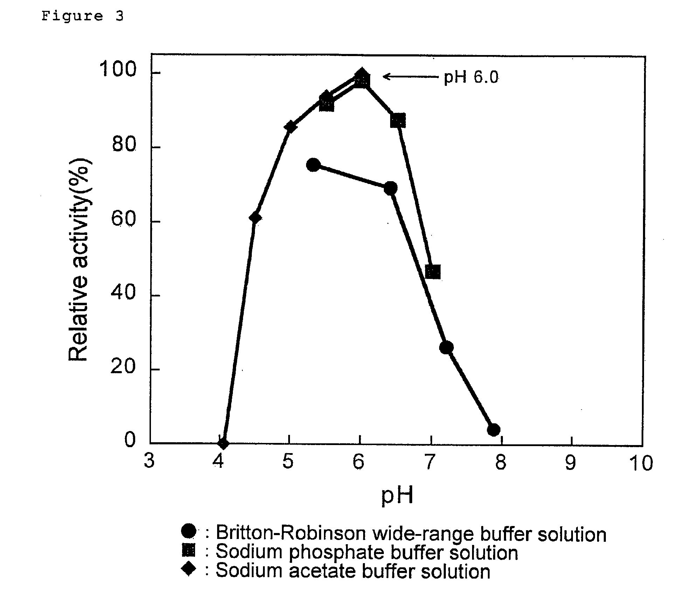

The (R)-1,3-butanediol oxidation activity and 4-hydroxy-2-butanone reduction activity of the enzyme obtained in Example 1 was investigated by changing the pH using potassium phosphate buffer solution, sodium acetate buffer solution, and Britton-Robinson wide-range buffer solution. The oxidation activity and the reduction activity are depicted in Figure 2 and 3, respectively, wherein the relative activity are indicated taking the maximum activity as 100. The optimum pH was 6.0 for both reactions, oxidation and reduction.

### [Example 4] Optimum temperature of the (R)-2,3-butanediol dehydrogenase

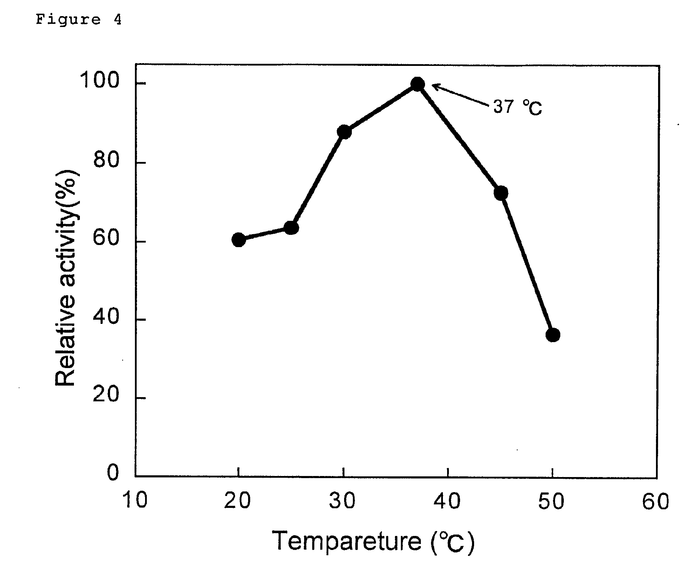

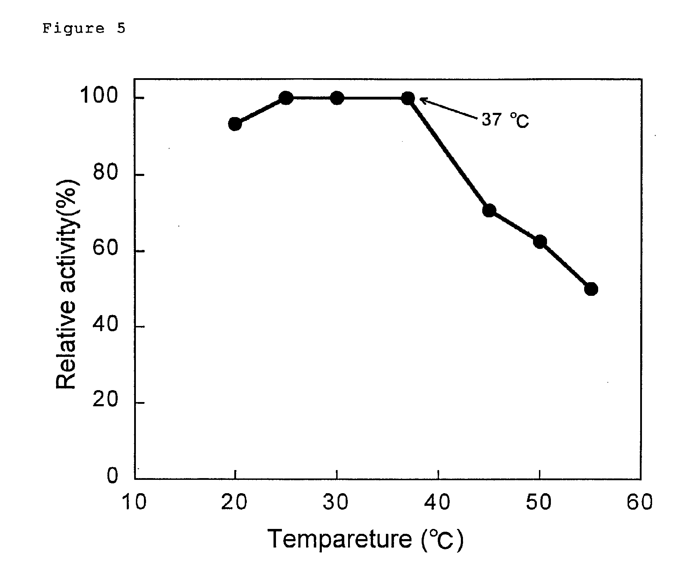

The (R)-1,3-butanediol oxidation activity and 4-hydroxy-2-butanone reduction activity of the enzyme obtained in Example 1 were measured by changing only the temperature of the standard reaction conditions. The oxidation activity and the reduction activity are depicted in Figure 4 and 5, respectively, wherein the relative activity is demonstrated taking the maximum activity as 100. The optimum reaction temperature was 37°C for both reactions, oxidation and reduction.

### [Example 5] Substrate Specificity of the (R)-2,3-Butanediol Dehydrogenase

The enzyme obtained in Example 1 was reacted with various alcohols and ketones, and the activity of oxidation and reduction is shown in Table 2. Herein, the relative activity is indicated taking the oxidation of (R)-1,3-butanediol and reduction of 4-hydroxy-2-butanone as 100.

**Table 2**

| Substrate | Coenzyme | Relative Activity |
|---|---|---|
| Oxidation Reaction | | |
| (R)-1,3-butanediol | NAD⁺ | 100 |
| (R) -2-butanol | NAD⁺ | 28 |
| Isopropyl alcohol | NAD⁺ | 48 |
| n-Butanol | NAD⁺ | 0 |
| Ethanol | NAD⁺ | 0 |
| Glycerin | NAD⁺ | 66 |
| (2R,3R)-butanediol | NAD⁺ | 15600 |
| (R)-1,2-propanediol | NAD⁺ | 13800 |
| (S)-3-chloro-1,2-propanediol | NAD⁺ | 128 |

| Reduction Reaction | | |
|---|---|---|
| 4-Hydroxy-2-butanone | NADH | 100 |
| 2-Butanone | NADH | 31 |
| Acetone | NADH | 63 |
| 3-Pentanone | NADH | 100 |
| 2,3-Butanedione | NADH | 46400 |
| 2,3-Pentanedione | NADH | 51400 |
| 2,4-Pentanedione | NADH | 0 |
| 4-Chloro-ethyl acetoacetate | NADH | 0 |
| Ethyl acetoacetate | NADH | 0 |
| Acetol | NADH | 53600 |
| Acetoin | NADH | 101000 |
| 1-Hydroxy-2-butanone | NADH | 12100 |
| Acetophenone | NADH | 36 |
| 2-Hydroxy-acetophenone | NADH | 360 |

### [Example 6] Stereoselectivity of the (R)-2,3-butanediol dehydrogenase

The enzyme obtained in Example 1 was reacted with 1,3-butanediol, 1,2-propanediol, 2,3-butanediol, 2-butanol, and 3-chloro-1,2-propanediol, and the activities are shown in Table 3. Herein, the relative activity is indicated, taking the higher oxidation activity between the two isomers as 100. The stereoselectivity for 2,3-butanediol, and 1,2-propandiol of the (R)-2,3-butanediol dehydrogenase according to the present invention was extremely high.

**Table 3**

| Substrate | Coenzyme | Relative Activity |
|---|---|---|
| (R)-1,3-butanediol (S)-1,3-butanediol | NAD⁺ NAD⁺ | 100 35 |
| (R)-2-butanol (S)-2-butanol | NAD⁺ NAD⁺ | 100 75 |
| (2R,3R)-2,3-butanediol (2S,3S)-2,3-butanediol | NAD⁺ NAD⁺ | 100 1 |
| (R)-1,2-propanediol (S)-1,2-propanediol | NAD⁺ NAD⁺ | 100 1 |
| (R)-3-chloro-1,2-propanediol (S)-3-chloro-1,2-propanediol | NAD⁺ NAD⁺ | 29 100 |

### [Example 7] Synthesis of the (R)-1,3-Butanediol Using (R)-2,3-Butanediol Dehydrogenase

2 mL reaction solution containing 200 mM potassium phosphate buffer solution (pH 6.5), 44 mg of NADH, 0.6 U of (R)-2,3-butanediol dehydrogenase, and 0.1% 4-hydroxy-2-butanone was reacted overnight at 25°C. The optical purity of the produced 1,3-butanediol was determined as follows. After saturating 2 mL reaction solution with sodium chloride, 1,3-butanediol was extracted with 2 mL ethyl acetate. After removing the extraction solvent, 100 µL acetyl chloride was added to the residue for acetylation. 1 mL of n-hexane was added to the resulting mixture to dissolve the acetylated 1,3-butanediol followed by analysis by liquid chromatography using an optical resolution column. The CHIRALCEL OB (4.6 mm x 25 cm) made by DAICEL Chemical Industries LTD. was used as the optical resolution column, and liquid chromatography was carried out with an elution solution of n-hexane:isopropanol= 19:1, a flow rate of 1.0 mL/min, monitoring at a wavelength of 220 nm, and a temperature of 40°C. As a result, the 1,3-butanediol produced according to the present invention was in the (R) conformation with an enatiomer excess of 99% ee or more.

Further, the resulting 1,3-butanediol was quantified by gas chromatography to determine the yield relative to the starting material, 4-hydroxy-2-butanone. The conditions for gas chromatography were as described below. Specifically, the gas chromatography was carried out using Porapak PS (Waters) column (mesh: 50-80, 3.2 mm x 210 cm) at a column temperature of 165°C, and the analysis was carried out using hydrogen flame ionization detector (FID). As a result, the yield of the reaction was determined to be about 95%.

### [Example 8] Partial Amino Acid Sequence of the (R)-2,3-Butanediol Dehydrogenase

The enzyme obtained in Example 1 was used to determine the N-terminal amino acid sequence with a protein sequencer. The amino acid sequence is shown in SEQ ID NO: 3. Further, a fragment containing the (R)-2,3-butanediol dehydrogenase was cut out from the SDS-PAGE gel, and after washing twice, in-gel digestion was carried out overnight at 35°C using lysyl endopeptidase. The digested peptide was separated and collected by a gradient elution with acetonitrile in 0.1% trifluoroacetic acid (TFA) using reverse phase HPLC (Tosoh TSK Gel ODS-80-Ts, 2.0 mm x 250 mm).

One of the collected peptide peak was dubbed lep_78, and the amino acid sequence was analyzed with protein sequencer (Hewlett Packard G1005A Protein Sequencer System). The amino acid sequence of lep_78 is shown in SEQ ID NO: 4.
SEQ ID NO: 3: N-terminal amino acid sequence
Met-Arg-Ala-Leu-Ala-Tyr-Phe-Gly-Lys-Gln
SEQ ID NO: 4: lep_78
Leu-Ala-Pro-Gly-Gly-Glu-Gly-Phe-Asp-Phe

### [Example 9] Purification of the Chromosomal DNA from Kluyveromyces lactis

*Kluyveromyces lactis* strain IFO 1267 was cultured in YM medium to prepare bacterial cells. Purification of the chromosomal DNA from the bacterial cells was performed according to the method described in Meth. Cell. Biol. 29, 39-44 (1975).

### [Example 10] Cloning of the Core Region of (R)-2,3-Butanediol Dehydrogenase Gene

A sense primer and an antisense primer were synthesized from the N-terminal and lep_78 amino acid sequences, respectively. The nucleotide sequences are shown in SEQ ID NO: 5 (KLB1-N) and SEQ ID NO: 6 (KLB1-78), respectively.

SEQ ID NO: 5: KLB1-N
GTCGGATCCGCHYTNGCHTAYTTYGGNAA

SEQ ID NO: 6: KLB1-78
CAGGGATCCRAARTCRAADCCYTCDCCDCC

Thirty cycles of denaturation (94°C, 30 seconds), annealing (50°C, 30 seconds) , and elongation (70°C, 40 seconds) were carried out using GeneAmp PCR System 2400 (Perkin-Elmer) with 50 µL reaction solution containing 50 pmol of each of the primer KLB1-N and KLB1-78, 10 nmol of dNTP, 50 ng of chromosomal DNA derived from *Kluyveromyces lactis,* Ex-Taq buffer solution (Takara Shuzo), and 2 U of Ex-Taq (Takara Shuzo).

According to an analysis by agarose gel electrophoresis of a portion of the PCR reaction solution, a band, predicted to be specific, was detected at about 800 bp. The DNA fragment was collected as a ethanol precipitate after extracting the fragment with phenol/chloroform. Then, the DNA fragment was digested with restriction enzyme *Bam*HI, subjected to agarose gel electrophoresis followed by cutting out the target band portion, and purification with Sephaglas Band Prep Kit (Pharmacia).

The resulting DNA fragment was ligated with pUC18, digested with *Bam*HI (Takara Shuzo), using Takara Ligation Kit, and then was used to transform *Escherichia coli* strain JM109.

The transformed strain was cultured on a plate of LB medium (containing 1% Bacto tryptone, 0.5% Bacto yeast extract, and 1% sodium chloride; hereinafter, abbreviated as LB medium) containing ampicillin (50 µg/mL), and several white colonies were selected according to the Blue/White selection method, and selected colonies were cultured in liquid LB medium containing ampicillin followed by purification of the plasmid with Flexi Prep (Pharmacia) to obtain pKLB1.

The nucleotide sequence of the inserted DNA was analyzed using the purified plasmid. PCR was carried out using BigDye Terminator Cycle Sequencing FS Ready Reaction Kit (Perkin-Elmer) on ABI PRISM™ 310 DNA sequencer (Perkin-Elmer) for analysis of the DNA nucleotide sequence. The determined nucleotide sequence of the core region is shown in SEQ ID NO: 7.

### [Example 12] Analysis of the nucleotide sequences in the vicinity of the core region of (R)-2,3-butanediol dehydrogenase gene

Chromosomal DNA derived from *Kluyveromyces lactis* was digested with restriction enzymes *Pst*I, *Hae*II, *Bam*HI, and *Nde*I, and cyclization of each fragment by a self-ligation reaction overnight at 16°C using T4 ligase was conducted. Next, 30 cycles of denaturation (94°C, 30 seconds), annealing (55°C, 30 seconds), and elongation (72°C, 7 minutes) were carried out using GeneAmp PCR System 2400 (Perkin-Elmer) with 50 µL of a reaction solution comprising 100 pmol of each of the primers KLB1-IP1 (SEQ ID NO: 8) and KLB1-IP2 (SEQ ID NO: 9), 25 ng of cyclic DNA, Ex-Taq buffer solution (Takara Shuzo), and 2 U of Ex-Taq (Takara Shuzo). As a result of analysis by agarose gel electrophoresis of a portion of the PCR reaction solution, DNA fragments of about 1600 bp, 4000 bp, 2400 bp, and 2100 bp were detected corresponding to the template DNA digested with *Pst*I, *Hae*II, *Bam*HI, and *Nde*I, respectively. These DNA fragments were then purified with Sephaglas Band Prep Kit (Pharmacia) and the nucleotide sequences were analyzed using KLB1-IP1 and KLB1-IP2. As a result, the ORF sequence of the (R)-2,3-butanediol dehydrogenase was determined. The determined DNA sequence is shown in SEQ ID NO: 1, and the predicted amino acid sequence encoded by the DNA is shown in SEQ ID NO: 2. The ORF search was carried out using Genetyx-win software (Software Development Co., Ltd.).

SEQ ID NO: 8: KLB1-IP1
CAGGGATCCACCGCAGATACCACACCATG
SEQ ID NO: 9; KLB1-IP2
GTCGGATCCGTGTCGAAACCTTCGATCCT

### [Example 13] Cloning of the (R)-2,3-butanediol dehydrogenase gene

Primers KLBDH1-N (SEQ ID NO: 10) and KLBDH1-C (SEQ ID NO: 11) were synthesized based on the structural gene sequence of the (R)-2,3-butanediol dehydrogenase for cloning the ORF alone. Thirty cycles of denaturation (95°C, 2 minutes 30 seconds), annealing (55°C, 1 minute), and elongation (72°C, 1 minute 30 seconds) were carried out using GeneAmp PCR System 2400 (Perkin-Elmer) with 50 µL reaction solution containing 50 pmol of each of the primers, 10 nmol of dNTP, 50 ng of chromosomal DNA derived from *Kluyveromyces lactis,* Pfu Turbo-DNA polymerase buffer (Stratagene), and 2.5 U of Pfu Turbo-DNA polymerase (Stratagene).

SEQ ID NO: 10: KLBDH1-N
CTTGAATTCTACCATGCGTGCATTAGCTTATTTCGG
SEQ ID NO: 11: KLBDH1-C
CAGCTTAAGATCTCTAGATTAGTTGGTGGCATCCAACTCACCATG

The obtained DNA fragment was extracted with phenol/chloroform to collect the DNA fragment as a ethanol precipitate. The DNA fragment was double-digested with restriction enzymes, *Eco*RI and *Afl*II, and was subjected to agarose gel electrophoresis. Then, the portion of the target band was cut out, and was purified with Sephaglas Band Prep Kit (Pharmacia).

The resulting DNA fragment was ligated with pSE420D (a plasmid vector, wherein the multi-cloning site of pSE420 (Invitrogen) has been modified, JP-A 2000-189170), double-digested with *Eco*RI and *Afl*II, using Takara Ligation Kit, followed by transformation of *Escherichia coli* strain HB101.

The transformed strain was grown on an LB medium plate containing ampicillin followed by analysis of the nucleotide sequence of the inserted fragment. The plasmid having the target (R)-2,3-butanedione dehydrogenase gene was dubbed pSE-KLB2.

### [Example 14]

### Production of the (R)-2,3-butanediol dehydrogenase by Escherichia coli

*Escherichia coli* strain HB101 transformed with the plasmid pSE-KLB2 that expresses the (R)-2,3-butanediol dehydrogenase was cultured overnight at 30°C in liquid LB medium containing ampicillin, followed by the addition of 0.1 mM IPTG, and culturing for an additional 4 hours.

After collecting the bacterial cells by centrifugation, the bacterial cells were suspended in 50 mM potassium phosphate buffer solution (pH 8.0) containing 0.02% 2-mercaptoethanol, 2 mM PMSF, and 10% glycerin, and then were homogenized by treating the cells for 3 minutes with closed ultrasonic disintegrator UCD-200TM (Cosmo Bio). The mixture containing homogenized bacterial cells was separated by centrifugation and the supernatant was collected as the cell extract. Furthermore, *Escherichia coli* having the plasmid pSE420D without the gene was cultured overnight in LB medium, followed by the addition of 0.1 mM IPTG, and culturing for an additional 4 hours. The resulting bacterial cells were homogenized in the same manner as described above followed by the measurement of the reduction activity on 2,3-butanedion. The results are shown in Table 4.

**Table 4**

| Plasmid | 2,3-Butanedion reduction activity |
|---|---|
| | U/mg-protein |
| None | 0.018 |
| pSE-KLB2 | 0.887 |

### [Example 15] Production of the (S)-1,3-butanediol with Escherichia coli strain HB101 transformed with pSE-KLB2

*Escherichia coli* strain HB101, that was transformed with pSE-KLB2, was cultured overnight at 30°C in 20 mL liquid LB medium containing ampicillin followed by the addition of 0.1 mM IPTG, and culturing for another 4 hours.

After collecting bacterial cells by centrifugation, the bacterial cells were suspended in 10 mL of 100 mM potassium phosphate buffer solution (pH 7.0) containing 1.0% racemic 1,3-butanediol, and were reacted with shaking overnight at 30°C. Following the reaction, the optical purity of the 1,3-butanediol in the reaction solution was analyzed. As a result, the remaining 1,3-butanediol was in the S conformation with an enatiomer excess of 99% ee or more.

Further, the 1,3-butanediol and 4-hydroxy-2-butanone in the reaction solution were quantified by gas chromatography. As a result, 4.9 g/L of 1,3-butanediol and 4.3 g/L of 4-hydroxy-2-butanone were determined to remain.

### [Example 16] Construction of plasmid pSF-KLB2 coexpressing the(R)-2,3-butanediol dehydrogenase gene and formic acid dehydrogenase gene derived from mycobacterium

Plasmid pSFR426 that expresses a formic acid dehydrogenase gene derived from *Mycobacterium* was double-digested with two restriction enzymes, *Nco*I and *Eco*RI, to prepare a DNA fragment containing the formic acid dehydrogenase gene derived from *Mycobacterium. E. coli* (JM109(pSFR426)) containing the plasmid has been deposited as follows.
Name and Address of Depositary Authority
Name: Patent and Bio-Resourse Center, National Institute of Advanced Industrial Science and Technology (AIST) (Previous Name: National Institute of Bioscience and Human-Technology, Agency of Industrial Science and Technology) Address: AIST Tsukuba Central 6, 1-1-3 Higashi, Tsukuba, Ibaraki, Japan
(ZIP CODE: 305-8566)
Date of Deposition (Date of Original Deposition): November 10, 2000
Accession Number: FERM BP-7392

pSE-KLB2 was double-digested with two restrictionenzymes, *Nco*I and *Eco*RI, and was ligated with the DNA fragment containing formic acid dehydrogenase gene derived from *Mycobacterium,* which fragment was cut out from pSFR426 with the same enzymes, using T4 DNA ligase to obtain plasmid pSF-KLB2 that simultaneously expresses the formic acid dehydrogenase and the (R)-2,3-butanediol dehydrogenase.

### [Example 17] Coexpression of the (R)-2,3-butanediol dehydrogenase and formic acid dehydrogenase by Escherichia coli

*Escherichia coli* strain HB101 transformed with pSF-KLB2 was cultured overnight at 30°C in liquid LB medium containing ampicillin followed by the addition of 0.1 mM IPTG, and culturing for another 4 hours.

After collecting the bacterial cells by centrifugation, the cells were suspended in 50 mM potassium phosphate buffer solution (pH 8.0) containing 0.02% 2-mercaptoethanol, 2 mM PMSF, and 10% glycerin. Then, the cells were treated for 3 minutes with closed ultrasonic disintegrator UCD-200TM (Cosmo Bio) to homogenize the cells. The homogenized bacterial cell liquid was separated by centrifugation and the supernatant was collected as the bacteria extract to measure the reduction activity on 2,3-butanedione and the formic acid dehydrogenation activity. Measurement of formic acid dehydrogenation activity was carried out at 30°C in a reaction solution containing 100 mM potassium phosphate buffer solution (pH 7.0), 2.5 mM NAD⁺, 100 mM formic acid, and the enzyme. 1 U was defined as the amount of enzyme that catalyzes the production of 1 µmol NADH in one minute under the above reaction conditions. As a result, the enzyme activity of the crude enzyme liquid obtained from *Escherichia coli* exhibited a 2,3-butanedione reduction activity of 0.665 U/mg-protein, and a formic acid dehydrogenase activity of 0.339 U/mg-protein.

### [Example 18] Production of (R)-1,3-butanediol by pSF-KLB2 Escherichia coli HB101

*Escherichia coli* strain HB101 transformed with pSF-KLB2 was cultured overnight at 30°C in liquid LB medium containing ampicillin followed by the addition of 0.1 mM IPTG and culturing for another 4 hours.

After collecting the bacterial cells by centrifugation, the bacterial cells were suspended in 10 mL of 500 mM potassium phosphate buffer solution (pH 7.0) containing 255 mM 4-hydroxy-2-butanone, and 511 mM sodium formate. Then, was reacted with shaking overnight at 30°C. After the reaction, the optical purity of produced 1,3-butanediol, as well as the amount of 1,3-butanediol and 4-hydroxy-2-butanone in the reaction solution were analyzed in the same manner as in Example 7. As a result, the optical purity of the produced (R)-1,3-butanediol was 99% *ee* or more and the reaction yield thereof was 65%.

### [Example 19] Inversion of configuration of racemic 1,3-butanediol using two kinds of bacterial cells

*Escherichia coli* strain HB101 which was transformed with plasmid pKK-CPA1, that expresses secondary alcohol dehydrogenase gene derived from *Candida parapucilosis* (JP-A Hei 7-231785), was cultured overnight at 30°C in 20 mL of liquid LB medium containing ampicillin followed by the addition of 0.1 mM IPTG and culturing for another 4 hours. The bacterial cells, dubbed bacterial cell A, were collected by centrifugation. Next, *Escherichia coli* strain HB101 transformed with pSF-KLB2 was cultured overnight at 30°C in 20 mL of liquid LB medium containing ampicillin followed by the addition of 0.1 mM IPTG and culturing for another 4 hours. The bacterial cells, dubbed bacterial cell B, were collected by centrifugation.

The bacterial cell A was suspended in 10 mL of 500 mM potassium phosphate buffer solution (pH 6.8) containing 443 mM racemic 1,3-butanediol and 20 g/L of yeast extract, and was reacted with shaking overnight at 30°C. After the reaction, 1,3-butanediol was in the R conformation with 95% *ee* and the concentration in the reaction solution was 203 mM, while the concentration of 4-hydroxy-2-butanone was 250 mM. After removing the bacterial cells from the reaction solution by centrifugation, the bacterial cell B and 500 mM sodium formate were added, and was reacted with shaking overnight at 30°C. After the reaction, 1,3-butanediol in the R conformation with 97.5% ee and the concentration in the reaction solution was 363 mM, and the concentration of 4-hydroxy-2-butanone was 22.7 mM. Thus, (R)-1,3-butanediol was recovered from racemic 1,3-butanediol with a yield of 82%.

## Claims

1. A purified (R)-2,3-butanediol dehydrogenase having the following physicochemical properties:
(1) Action: the enzyme acts on (2R,3R)-2,3-butanediol, using nicotinamide adenine dinucleotide (hereinafter, abbreviated as NAD⁺) as a coenzyme, to produce (R)-acetoin and reduces 2,3-butanedione, using the reduced form of nicotinamide adenine dinucleotide (hereinafter, abbreviated as NADH) as a coenzyme, to produce (2R,3R)-2,3-butanediol;
(2) Substrate specificity:
(a) the enzyme uses NAD⁺ as a coenzyme in the oxidation reaction and NADH as a coenzyme in the reduction reaction;
(b) the enzyme reduces 4-hydroxy-2-butanone to produce (R)-1,3-butanediol;
(c) the enzyme preferentially oxidizes the hydroxyl group of the (R) configuration of racemic 1,3-butanediol to remain the (S)-1,3-butanediol; and
(3) Optimal pH of 6.0 for both of the oxidation reaction and the reduction reaction.

2. The (R)-2,3-butanediol dehydrogenase of claim 1, wherein the enzyme further has the following additional physicochemical properties:
(4) an optimum temperature of 37°C for both of the oxidation reaction and reduction reaction; and
(5) a molecular weight determined by sodium dodecyl sulfate-polyacrylamide gel electrophoresis (hereinafter, abbreviated as SDS-PAGE) and by gel filtration of 45,000 and 91,000, respectively.

3. The (R)-2,3-butanediol dehydrogenase of claim 1, wherein the enzyme is produced by and isolated from a microorganism belonging to the genus *Kluyveromyces.*

4. The (R)-2,3-butanediol dehydrogenase of claim 3, wherein the microorganism is *Kluyveromyces lactis.*

5. An isolated polynucleotide selected from the group consisting of:
(a) a polynucleotide comprising the nucleotide sequence of SEQ ID NO: 1;
(b) a polynucleotide encoding a polypeptide comprising the amino acid sequence of SEQ ID NO: 2;
(c) a polynucleotide encoding a polypeptide comprising the amino acid sequence of SEQ ID NO: 2 in which one or more amino acids are substituted, deleted, inserted, and/or added, wherein said polypeptide is functionally equivalent to a polypeptide consisting of the amino acid sequence of SEQ ID NO: 2;
(d) a polynucleotide hybridizing under stringent conditions with a DNA consisting of the nucleotide sequence of SEQ ID NO: 1, wherein the polypeptide encoded by the polynucleotide is functionally equivalent to the polypeptide consisting of the amino acid sequence of SEQ ID NO: 2; and
(e) a polynucleotide encoding an amino acid sequence having 80% or more identity with the amino acid sequence of SEQ ID NO: 2, wherein the polypeptide encoded by the polynucleotide is functionally equivalent to the polypeptide consisting of the amino acid sequence of SEQ ID NO: 2.

6. A purified polypeptide encoded by the polynucleotide of claim 5.

7. A vector comprising the polynucleotide of claim 5.

8. A transformant retaining the polynucleotide of claim 5 or the vector of claim 7.

9. A method for producing a polypeptide encoded by the polynucleotide of claim 5, comprising the steps of culturing a transformant that retains the polynucleotide of claim 5 or a vector that comprises the polynucleotide of claim 5, and recovering the expressed product thereof.

10. A method for producing the purified enzyme of claim 1 or the purified polypeptide of claim 6, comprising the step of culturing a microorganism belonging to the genus *Kluyveromyces*, that produces the enzyme of claim 1 or the polypeptide of claim 6, and extracting and purifying the enzyme or polypeptide produced from the culture.

11. The method of claim 10, wherein the microorganism is *Kluyveromyces lactis.*

12. A method for producing an optically active alcohol, comprising the steps of reacting a ketone in the presence of NADH with any enzyme active substance selected from the group consisting of: (a) the (R)-2,3-butanediol dehydrogenase of claim 1, (b) the polypeptide of claim 6, (c) a microorganism producing (a) or (b), and (d) processed products of (a), (b) or (c); and obtaining the optically active alcohol produced by the reduction of the ketone.

13. The method of claim 12, wherein the microorganism is the transformant of claim 8.

14. The method of claim 12, wherein the ketone is 4-hydroxy-2-butanone, and the optically active alcohol is (R)-1,3-butanediol.

15. A method for producing optically active alcohols, comprising the steps of reacting a racemic alcohol in the presence of NAD⁺ with any enzyme active substance selected from the group consisting of: (a) the (R)-2,3-butanediol dehydrogenase of claim 1, (b) the polypeptide of claim 6, (c) a microorganism that produces (a) or (b), and (d) processed products of (a) to (c), to oxidize one of the optical isomers; and obtaining the remaining optically active alcohol.

16. The method of claim 15, wherein the microorganism is the transformant of claim 8.

17. The method of claim 15, wherein the racemic alcohol is racemic 1,3-butanediol, and the optically active alcohol is (S)-1,3-butanediol.

18. A method for producing ketones comprising the steps of contacting an alcohol in the presence of NAD⁺ with any enzyme active substance selected from the group consisting of: (a) the (R)-2,3-butanediol dehydrogenase of claim 1, (b) the polypeptide of claim 6, (c) a microorganism that produces (a) or (b), and (d) processed products of (a) to (c); and obtaining the ketone produced by the oxidation of the alcohol.

19. The method of claim 18, wherein the microorganism is the transformant of claim 8.

20. The method of claim 18, wherein the alcohol is (R)-1,3-butanediol, and the ketone is 4-hydroxy-2-butanone.

21. A method for producing (R)-1,3-butanediol, comprising the steps of:
(1) producing (R)-1,3-butanediol and 4-hydroxy-2-butanone by preferentially oxidizing (S)-1,3-butanediol by reacting racemic 1,3-butanediol with any enzyme active substance selected from the group consisting of: an enzyme that produces 4-hydroxy-2-butanone, a microorganism producing the enzyme, and processed products of the enzyme or microorganism;
(2) producing (R)-1,3-butanediol by reducing 4-hydroxy-2-butanone by contacting the 4-hydroxy-2-butanone produced in step (1) with any enzyme active substance selected from the group consisting of: an enzyme that produces (R)-1,3-butanediol, a microorganism producing the enzyme, and processed products of the enzyme or microorganism; and
(3) obtaining the (R)-1,3-butanediol produced in step (2).

22. The method of claim 21, wherein the enzyme active substance in the above step (2) is any enzyme active substance selected from the group consisting of: the (R)-2,3-butanediol dehydrogenase of claim 1; the polypeptide of claim 6; a microorganism that produces either or both of them; and processed products thereof.

23. The method of claim 21, wherein the enzyme active substance in the above step (1) is **characterized** as being a microorganism or processed product thereof that produces (S) conformation-specific secondary alcohol dehydrogenase derived from *Candida parapucilosis.*

24. The polynucleotide of claim 5, wherein the modification of (c) comprises conservative amino acid modification, such that the modified amino acid retains the amino acid side-chain properties of the original amino acid.

25. The polynucleotide of claim 5, wherein the modified polypeptide of (c) has the amino acid sequence of SEQ ID NO: 15.

26. The polynucleotide of claim 5, wherein the modification by addition of (c) results in a fusion protein.

27. The polynucleotide of claim 5, wherein the number of amino acids modified by substitution, deletion, insertion and/or addition is less than 50.

28. The polynucleotide of claim 5, wherein the number of amino acids modified by substitution, deletion, insertion and/or addition is less than 20.

29. The polynucleotide of claim 5, wherein the number of amino acids modified by substitution, deletion, insertion and/or addition is less than 5.

30. The polynucleotide of claim 5, wherein the polynucleotide encodes an enzyme possessing the properties recited in claim 1 and having at least 50% sequence identity with the polypeptide consisting of the amino acid sequence of SEQ ID NO: 2.

31. The polynucleotide of claim 5, wherein the polynucleotide encodes an enzyme possessing the properties recited in claim 1 and having at least 70% sequence identity with the polypeptide consisting of the amino acid sequence of SEQ ID NO: 2.

32. The polynucleotide of claim 5, wherein the polynucleotide encodes an enzyme possessing the properties recited in claim 1 and having at least 90% sequence identity with the polypeptide consisting of the amino acid sequence of SEQ ID NO: 2.

33. The polynucleotide of claim 5, wherein the polynucleotide encodes an enzyme possessing the properties recited in claim 1 and having at least 95% sequence identity with the polypeptide consisting of the amino acid sequence of SEQ ID NO: 2.
